# FASCICULE DE BREVET EUROPEEN

(11) **EP 1 729 720 B1**
(45) Date de publication et mention de la délivrance du brevet: **19.11.2008**
(21) Numéro de dépôt: 05732925.2
(22) Date de dépôt: 25.02.2005
(51) Int. Cl.: A61K 8/81

(54) **COMPOSITION COMPRENANT UN POLYMERE COMPRENANT UN COMPOSE MONOMERIQUE A EFFET OPTIQUE, PROCEDE EMPLOYANT LADITE COMPOSITION**
ZUSAMMENSETZUNG ENTHALTEND EIN POLYMER BASIEREND AUF EINER MONOMERVERBINDUBG GEKENNZEICHNET DURCH EINE OPTISCHE EINGENSCHAFT UND EIN VERFAHREN ZU DEREN VERVENDUNG
COMPOSITION COMPRISING A POLYMER COMPRISING A MONOMER COMPOUND EXHIBITING AN OPTICAL PROPERTY, METHOD MAKING USE OF SAID COMPOSITION

(30) Priorité: 23.03.2004 FR 0403004; 23.03.2004 FR 0403002; 07.04.2004 US 560016 P; 09.04.2004 US 560704 P
(43) Date de publication de la demande: 13.12.2006
(73) Titulaire: L'ORÉAL, 75008 Paris (FR)
(72) Inventeur: LUUKAS, Timo, F-91300 Massy (FR)
(74) Mandataire: Dodin, Catherine
(86) Numéro de dépôt international: PCT/FR2005/000458
(87) Numéro de publication internationale: WO 2005/102249

(56) Documents cités:
- EP-A- 0 728 745
- EP-A- 0 767 234
- US-A- 5 626 839
- US-B1- 6 645 428
- YAN XIAO-LI ET AL: "Fluorescence property of two naphthalimide derivatives and their polymers" CAPLUS, 2000, XP002203163

## Description

La présente invention a trait à de nouvelles compositions cosmétiques ou pharmaceutiques, notamment à application topique, et notamment de nouvelles compositions de maquillage, comprenant des polymères organiques présentant des propriétés optiques, notamment de fluorescence, particulières.

Les compositions cosmétiques, et notamment les compositions de maquillage telles que les poudres libres ou compactes, les fonds de teint, les fards à joues ou à paupières, les rouges à lèvres ou les vernis à ongles, sont généralement constituées d'un véhicule approprié et d'un ou plusieurs agents de coloration destinés à conférer une certaine couleur auxdites compositions avant et/ou après leur application sur la peau, les muqueuses, les semi-muqueuses et/ou les phanères telles que les ongles, les cils ou les cheveux.
Pour créer des couleurs, on utilise aujourd'hui une gamme d'agents de coloration assez limitée comprenant notamment des laques, des pigments minéraux, des pigments organiques et des pigments nacrés. Les pigments et laques utilisés dans le domaine du maquillage sont d'origine et de nature chimique très diverses. Leurs propriétés physico-chimiques, notamment granulométrie, surface spécifique, densité, etc., sont donc très différentes. Ces différences se traduisent par des variations de comportement : leur facilité de mise en oeuvre, de dispersion dans le milieu; leur stabilité à la lumière, à la température; leurs propriétés mécaniques. Les pigments minéraux, en particulier les oxydes minéraux sont au contraire très stables à la lumière et au pH mais donnent des couleurs plutôt ternes et pâles. Il est donc nécessaire d'en introduire une grande quantité dans les formulations cosmétiques pour obtenir un trait suffisamment saturé. Ce fort pourcentage de particules minérales peut néanmoins affecter la brillance de la composition. Les pigments nacrés quant à eux permettent d'obtenir des couleurs variées mais peu intenses, qui conduisent à des effets irisés mais le plus souvent assez faibles. Dans le domaine de la coloration capillaire temporaire ou fugace, qui donne lieu à une modification légère de la couleur naturelle de la chevelure qui tient d'un shampooing à l'autre et qui sert à embellir ou corriger une nuance déjà obtenue, on a déjà proposé une coloration avec des pigments usuels pour apporter un reflet temporaire aux cheveux, mais les nuances obtenues par cette coloration restent assez ternes, trop uniformes et peu ludiques.
Dans le domaine du maquillage, seules les laques organiques permettaient jusqu'à présent d'obtenir des couleurs vives et intenses. Cependant, la plupart des laques organiques présentent une très mauvaise tenue à la lumière, qui se traduit par une atténuation très nette de leur couleur dans le temps. Elles peuvent également être instables à la température et/ou au pH. De plus, certaines laques génèrént un dégorgement trop important, c'est-à-dire qu'elles présentent l'inconvénient de tacher le support sur lequel elles sont appliquées. Ainsi, ceci peut avoir pour conséquence de tâcher les lentilles oculaires dans le cas des eye-liners ou des mascaras, ou de laisser une coloration sur la peau ou les ongles après démaquillage dans le cas des rouges à lèvres ou des vernis à ongles. Enfin, l'instabilité des laques est encore aggravée lorsqu'elles sont associées à des pigments photoréactifs comme le dioxyde de titane. Or ces pigments sont très largement utilisés dans le maquillage, notamment pour la protection contre le rayonnement UV. Par conséquent, l'utilisation des laques organiques en cosmétique est assez limitée, ce qui a pour conséquence une limitation des teintes réalisables.

Ainsi il subsiste le besoin de disposer de polymères organiques à propriétés optiques, susceptibles d'être utilisés en cosmétique, permettant d'obtenir des effets optiques adéquats des compositions les comprenant et/ou du maquillage obtenu à l'aide de ces compositions, lesdits polymères ayant par ailleurs une bonne stabilité thermique et photochimique, tout en présentant un faible dégorgement.
Après de nombreuses recherches, la demanderesse a mis en évidence que l'utilisation d'une famille bien précise de polymères, comprenant en fait au moins un monomère bien particulier, permettait d'obtenir de manière inattendue, un tel résultat.

Ainsi l'invention a pour objet une composition cosmétique ou pharmaceutique comprenant, dans un milieu physiologiquement acceptable, au moins un polymère comprenant au moins un composé monomérique tel que défini ci-après.

Un autre objet de l'invention est un procédé cosmétique de maquillage ou de soin des matières kératiniques, notamment de la peau du corps ou du visage, des lèvres, des ongles, des cils, des sourcils et/ou des cheveux, comprenant l'application sur lesdites matières d'une telle composition cosmétique.

Les polymères utilisés, selon l'invention peuvent se présenter sous forme solide ou liquide, et confèrent des effets optiques remarquables aux compositions qui les comprennent ainsi qu'au maquillage déposé; en particulier, ils peuvent apporter des effets éclaircissants ou de couleur.
Ces effets optiques peuvent être avantageusement modulés en fonction de la nature chimique et/ou de la position des différents substituants présents sur le monomère à effet optique employé pour former le polymère. D'une manière générale, lorsque le groupement X est un oxygène, le monomère résultant sera plutôt de couleur jaune/orange; lorsque le groupement X comprend un atome d'azote, le monomère résultant sera plutôt dans le domaine du rouge.

Parmi les autres avantages que peuvent procurer ces polymères, on peut noter leur bonne stabilité à la température, au pH et à la lumière.

On a également constaté que les polymères utitisés selon l'invention présentaient une bonne solubilité dans les corps gras, solubilité qui pouvait varier et être ajustée, selon la nature des monomères. Cette bonne liposolubilité peut également faciliter leur mise en oeuvre ultérieure, notamment dans les compositions cosmétiques qui comprennent généralement une phase grasse.
De plus, les bonnes propriétés cosmétiques des compositions selon l'invention sont maintenues, lorsqu'elles comprennent ces polymères selon l'invention.

De plus, bien que de structure chimique proche, les polymères selon l'invention peuvent présenter, selon la nature des substituants, une grande variété d'effets optiques, pouvant aller du jaune au rouge/violet. Ceci permet de disposer d'une gamme de composés, appartenant à la même famille chimique, et donc se formulant de manière similaire, qui proposent des diversités de couleurs ou de propriétés optiques remarquables; ceci facilite notamment le travail des formulateurs en leur permettant de garder une architecture commune à l'ensemble de leurs compositions, quel que soit les polymères à propriété optique employés.

Par ailleurs, on a constaté que les monomères (I) définis ci-après et les polymères les comprenant, présentent de bonnes propriétés de fluorescence. On rappelle que les composés fluorescents absorbent dans l'ultraviolet et dans le visible, et réémettent de l'énergie par fluorescence pour une longueur d'onde comprise entre 380 nm et 830 nm.

En outre, les polymères présentent l'avantage de se démaquiller aisément.

La composition selon l'invention comprend donc, dans un milieu physiologiquement acceptable, notamment un milieu cosmétiquement ou pharmaceutiquement acceptable, au moins un polymère susceptible d'être obtenu par polymérisation notamment radicalaire d'au moins un monomère de formule (I).

Ledit monomère de formule (I) répond donc à la formule suivante : 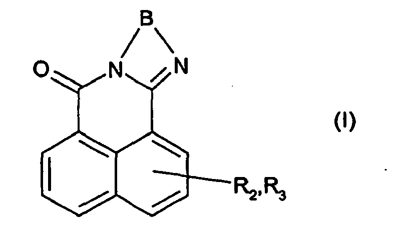 dans laquelle :
- R2 et R3, présents sur le même cycle ou chacun sur un cycle différent, représentent, indépendamment l'un de l'autre, un hydrogène, un halogène, ou un groupement de formule -X-G-P (II), sous réserve qu'au moins l'un des radicaux R2 et/ou R3 représente un groupement de formule (II), dans laquelle :
- X est choisi parmi les groupements -O-, -S-, -SO-, -SO₂-, -NH- ou -NR- avec R représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- G est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- P est un groupement polymérisable choisi parmi l'une des formules suivantes : 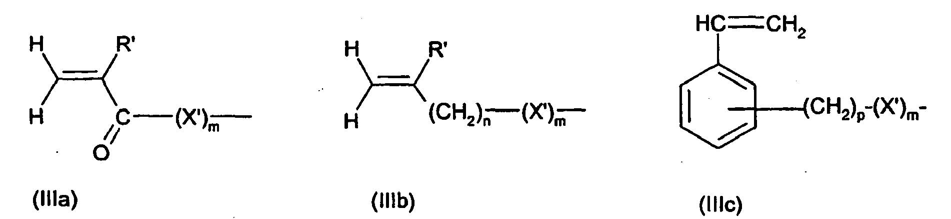 dans lesquelles :
- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyles et/ou aryles pouvant en outre être substitués par un ou plusieurs groupements choisis parmi OH, halogène, alcoxy en C1-6 et aryloxy en C6-10; de préférence X' représente O;
- m est égal à 0 ou 1; n est égal à 0 ou 1; p est égal à 0, 1 ou 2.
- B représente l'un des groupements aromatiques divalents suivants (IVa) à (IVd) : 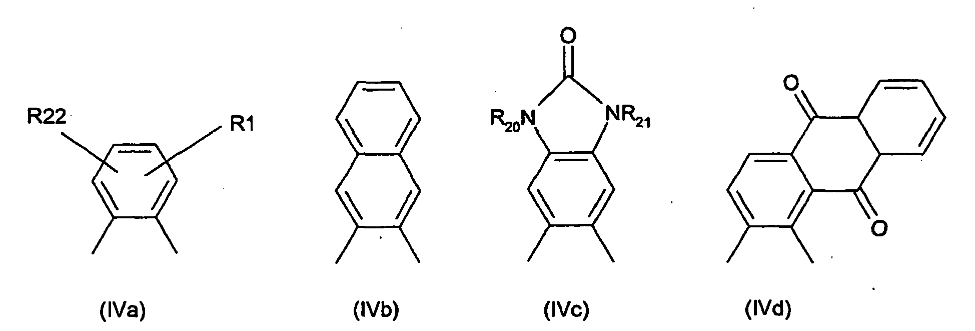 dans lesquels :
- R1 est un radical carboné linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S.
- R22 est un atome d'hydrogène ou un radical carboné linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- R20 et R21 sont, indépendamment l'un de l'autre, un atome d'hydrogène, un radical alkyle linéaire ou ramifié en C1-8, un radical cyclopentyle, cyclohexyle, cyclooctyle, cyclodécyle, cyclododécyle, benzyle, naphtyle ou phényle.

On notera que certains des composés pour lesquels, simultanément, P est de formule (IIIa), X' est O, m=1, X est NH et B est de formule (IVc), peuvent être connus.

Dans la présente invention, on entend par 'radical cyclique' un radical monocyclique ou polycyclique, qui se présente donc lui-même sous forme d'un ou plusieurs cycles, saturés et/ou insaturés, éventuellement substitués (par exemple cyclohexyle, cyclodécyle, benzyle ou fluorényle), mais également un radical qui comprend un ou plusieurs desdits cycles (par exemple p-tertbutylcyclohexyle ou 4-hydroxybenzyle).

Dans la présente invention, on entend par 'radical saturé et/ou insaturé', les radicaux totalement saturés, les radicaux totalement insaturés, y compris aromatiques, ainsi que les radicaux comportant une ou plusieurs doubles et/ou triples liaisons, le reste des liaisons étant des liaisons simples.

De préférence, R2 est un atome d'hydrogène, et donc R3 un groupement de formule (II).

Dans ledit groupement de formule (II), X est de préférence choisi parmi -O-, -NH-et -NR- avec R représentant préférentiellement un radical hydrocarboné linéaire, ramifié et/ou cyclique, saturé ou insaturé, comprenant éventuellement un cycle hydrocarboné lui-même saturé ou insaturé, comprenant 2 à 18, notamment 3 à 12 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
Notamment R peut être un radical éthyle, n-propyle, isopropyle, n-butyle, isobutyle, tert-butyle, pentyle, hexyle, cyclohexyle, octyle, cyclooctyle, décyle, cyclodécyle, dodécyle, cyclododécyle, phényle ou benzyle.
Préférentiellement, X est choisi parmi -NH- et -NR- avec R représentant un cyclohexyle.

Toutefois, lorsque X est égal à NR, alors B est de préférence différent de la formule (IVa).

Le radical divalent G est de préférence un radical hydrocarboné divalent linéaire, ramifié et/ou cyclique, saturé ou insaturé, comprenant éventuellement un cycle hydrocarboné lui-même saturé ou insaturé, comprenant au total 2 à 18, notamment 3 à 10 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si. Préférentiellement G est choisi parmi les radicaux hydrocarbonés divalents linéaires ou ramifiés, saturés comprenant éventuellement un cycle hydrocarboné saturé, comprenant au total 2 à 18, notamment 3 à 10 atomes de carbone.
Ainsi G peut être choisi parmi les radicaux éthylène, n-propylène, isopropylène (ou méthyl-1 éthylène et méthyl-2 éthylène), n-butylène, isobutylène, pentylène notamment n-pentylène, hexylène notamment n-hexylène, cyclohexylène, heptylène, octylène, cyclooctylène, décylène, cyclodécylène, cyclohexyldiméthylène notamment de formule -CH₂-C₆H₁₀-CH₂-, dodécylène, cyclododécylène.

Dans la formule (IIIb), si n =0 alors de préférence, m=0.

Le groupement polymérisable P est de préférence choisi parmi l'une des formules suivantes : 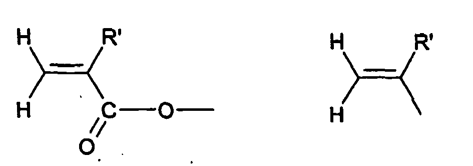 dans lesquelles R' représente H ou méthyle.

Le groupement B est de préférence choisi parmi ceux de formule (IVa) dans laquelle R1 est préférentiellement un radical carboné linéaire, ramifié et/ou cyclique, saturé, comprenant 1 à 32 atomes de carbone, notamment 2 à 12, voire 3 à 6 atomes de carbone; en particulier R1 peut être un radical méthyle, éthyle ou propyle.

Parmi les composés monomériques particulièrement préférés selon l'invention, on peut citer les composés répondant à l'une des formules suivantes, dans lesquelles R est l'hydrogène ou méthyle : 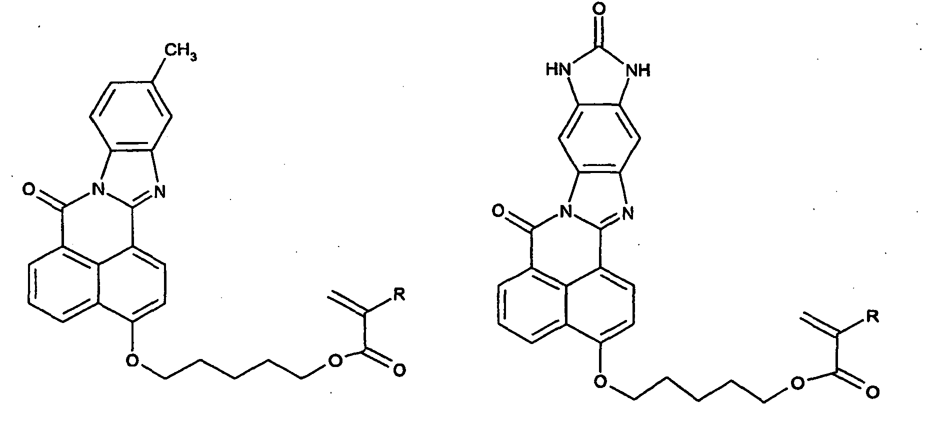 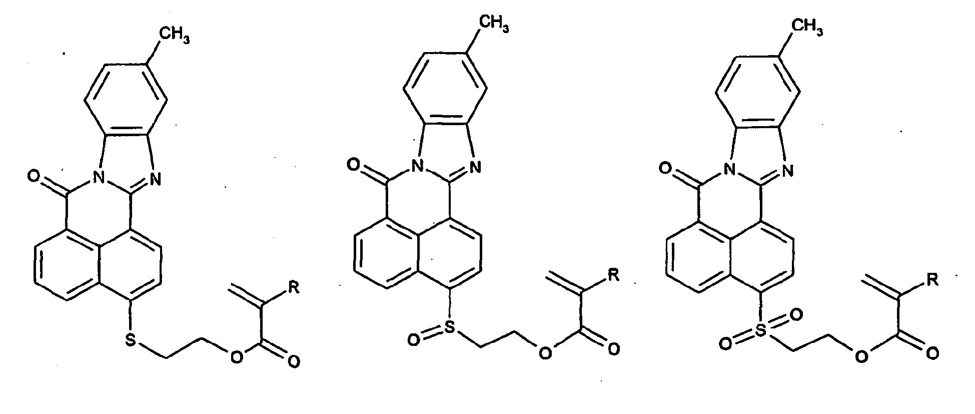 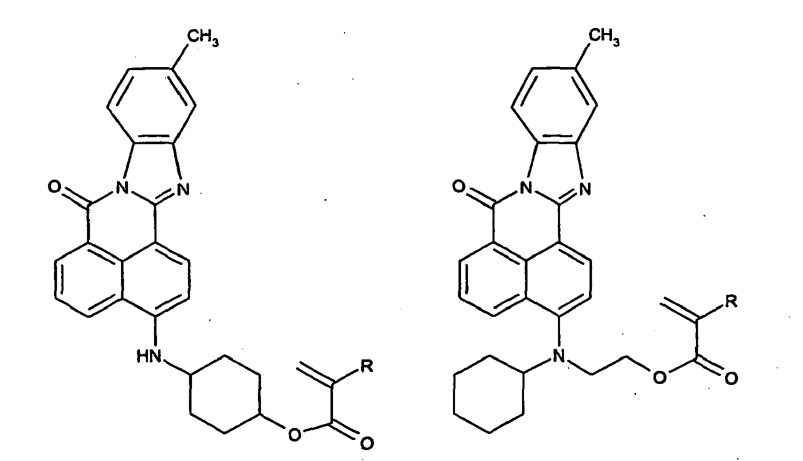 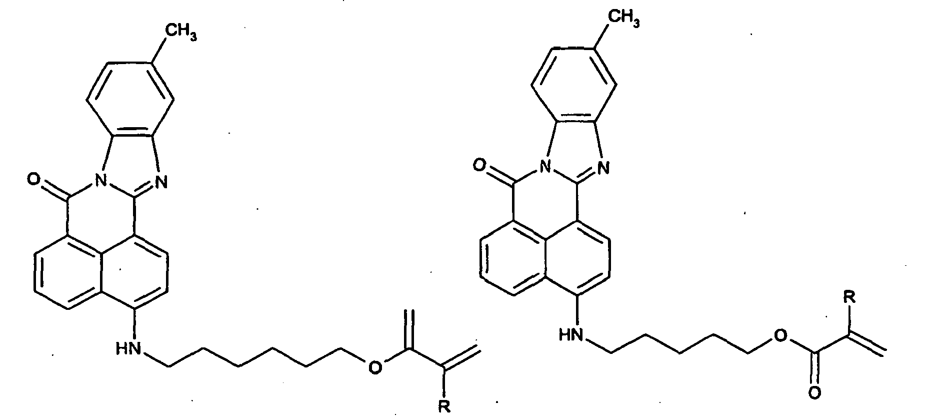 Les monomères (I) et les polymères les comprenant, présentent de bonnes propriétés optiques et sont susceptibles d'être préparés plus aisément que ceux de l'art antérieur.
Notamment en vue d'une exploitation industrielle, on recherche des composés, monomères et polymères, dont la réactivité est élevée ce qui permet un temps de réaction (polymérisation) court.
On recherche également des monomères et polymères présentant de bonnes propriétés optiques, avec une palette de couleur étendue, et susceptibles d'être employés en cosmétique.
On a constaté qu'avec les composés selon la présente invention, la polymérisation est plus aisée, notamment de par la présence d'un groupement espaceur (G).
En outre, les polymères et composés monomérique mis en oeuvre selon l'invention trouvent une utilisation toute particulière pour conférer à une composition des effets optiques, notamment de fluorescence.

Certains de ces composés peuvent notamment être préparés selon l'état de la technique, par exemple selon l'enseignement du document EP728745, en particulier les composés pour lesquels X est N.

D'une façon schématique, le procédé général de synthèse, pour les composés pour lesquels X est O ou S, peut être représenté comme suit:

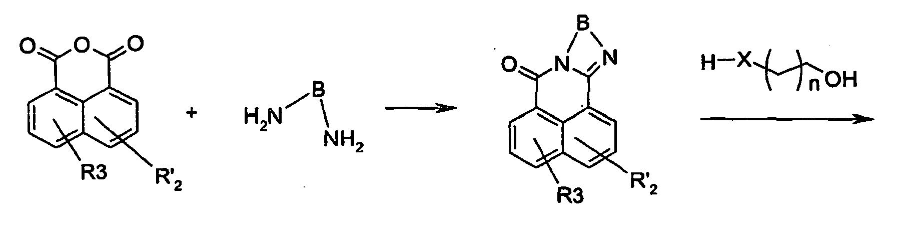

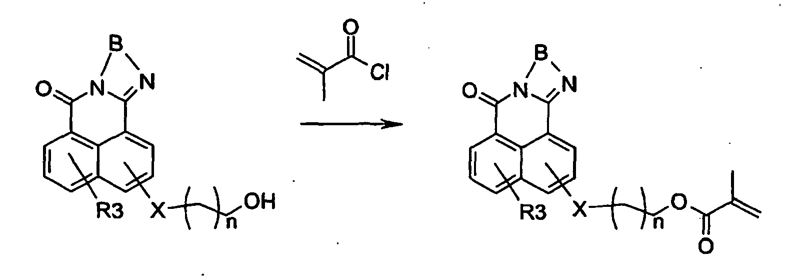

On peut ainsi faire réagir l'anhydride naphthalique adéquat avec une diamine primaire adéquate.
De préférence, la diamine est présente en léger excès par rapport à l'anhydride naphthalique, notamment à raison de 1 à 1,5 équivalent, de préférence 1,1 équivalent, pour 1 équivalent d'anhydride.
La réaction peut être effectuée dans un solvant choisi parmi les solvants dans lequel l'anhydride est soluble, et notamment le toluène, le xylène, l'acide acétique ou le NMP; la réaction est de préférence effectuée à reflux du solvant, par exemple à une température de 50-250°C, de préférence 80-160°C.

Puis on peut faire réagir l'isoquinolinone formé avec un diol, un aminoalcool ou un thioalcool.
Par exemple, lorsque R'2 est un halogène (chlore ou brome de préférence), il est possible d'effectuer une substitution nucléophile aromatique, en employant par exemple un diol ou un thioalcool, tels que le 1,3-propane-diol, le 1,5-propane-diol ou le 2-mercaptoéthanol, éventuellement sous forme d'alcoolate de métal alcalin (sodium par exemple).
La réaction peut être effectuée en l'absence de solvant, ou en présence d'un solvant dipolaire aprotique tel que le dichlorométhane, le THF (tétrahydrofurane), notamment à une température de 20-150°C.

Les dérivés soufrés peuvent être oxydés dans des conditions douces de manière à conduire au sulfoxyde correspondant. En modifiant les conditions de l'oxydation, il est également possible de préparer le sulfone correspondant. On peut ensuite transformer ces sulfides, sulfoxydes et sulfones afin d'obtenir les méthacrylates ou acrylates recherchés.

Les dérivés alcooliques, quant à eux, peuvent être réagis avec un halogénure de (méth)acryloyle, notamment un chlorure, de manière à former le (méth)acrylate correspondant.
Cette réaction peut être effectuée en présence d'une base telle que la triéthanolamine, dans un solvant tel que le tetrahydrofurane ou le dichlorométhane, notamment à une température de -30°C à 100°C, de préférence 0 à 80°C.

Ces composés monomériques peuvent être utilisés comme premier monomère pour préparer des copolymères les comprenant.

En particulier, les composés monomériques à effet optique utilisés selon l'invention peuvent être employés pour préparer des homopolymères ou des copolymères ne comprenant que des composés monomériques à effet optique de formule (I), seul ou en mélange, ou bien de formule (I) en mélange avec d'autres, notamment en mélange avec ceux de formule A et/ou B telles que définies ci-après, ces différents composés pouvant alors par exemple être présents chacun à raison de 0,5 à 99,5% en poids, notamment 5 à 95% en poids, voire 10 à 90% en poids, encore mieux chacun à raison de 30 à 70% en poids, par rapport au poids total du polymère. Ceci peut permettre notamment de préparer des polymères présentant une large palette d'effet optique (couleur notamment, azurant optique ou autre).

Parmi les composés monomériques à effet optique susceptibles d'être copolymérisés avec les composés monomériques de formule (I), et éventuellement avec un ou plusieurs des comonomères additionnels tels que définis ci-après, on peut citer les composés de formule (A) et/ou de formule (B) ci-après : 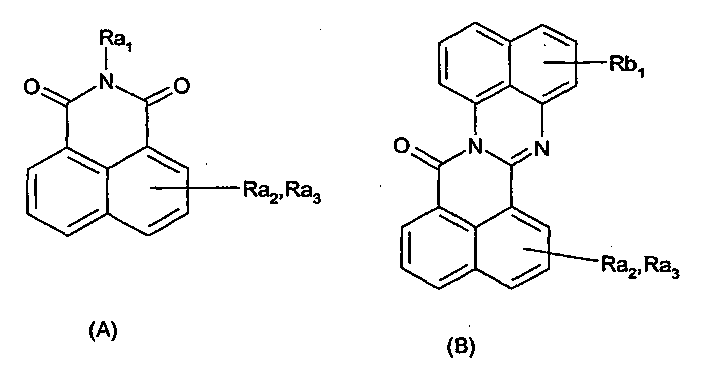 dans laquelle :
- Ra1 représente un radical carboné linéaire, ramifié et/ou cyclique, saturé et/ou insaturé, comprenant 1 à 32 atomes de carbone; éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- Rb1 est choisi parmi (i) un atome d'hydrogène, (ii) un halogène, (iii) un radical carboné linéaire, ramifié et/ou cyclique, saturé et/ou insaturé, comprenant 1 à 12 atomes de carbone, éventuellement substitué par un ou plusieurs groupements choisis parmi =O, OH, NH₂ et/ou éventuellement interrompu par un ou plusieurs hétéroatomes choisis parmi O, N, P, Si et S; (iv) un groupement NRR' avec R et R' étant, indépendamment 'un de l'autre, un atome d'hydrogène ou un radical hydrocarboné, linéaire, cyclique ou ramifié, saturé en C1-6, notamment méthyle, éthyle, propyle, isopropyle, n-butyle, iso-butyle, tert-butyle, pentyle ou hexyle;
- Ra2 et Ra3, présents sur le même cycle ou chacun sur un cycle différent, représentent, indépendamment l'un de l'autre, un hydrogène, un halogène, ou un groupement de formule -Xa-Ga-Pa (II), sous réserve qu'au moins l'un des radicaux Ra2 et/ou Ra3 représente un groupement de formule (II), dans laquelle :
- Xa est choisi parmi les groupements -O-, -S-, -SO-, -SO₂-, -NH- ou -NR4- avec R4 représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- Ga est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- Pa est un groupement polymérisable choisi parmi l'une des formules suivantes : 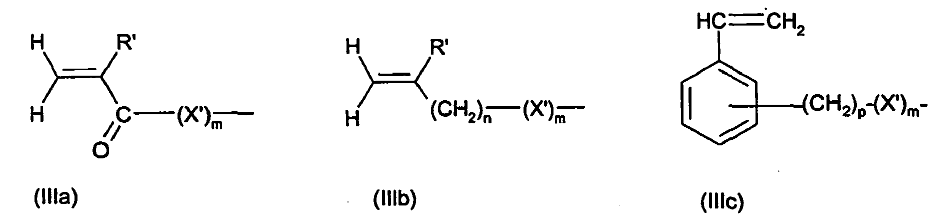 dans lesquelles :
- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyles et/ou aryles pouvant en outre être substitués par un ou plusieurs groupements choisis parmi OH, halogène, alcoxy en C1-6 et aryloxy en C6-10; et
- m est égal à 0 ou 1;n est égal à 0 ou 1; p est égal à 0, 1 ou 2.

Les copolymères utilisés selon l'invention peuvent être statistiques, alternés ou greffés, ou séquencés, par exemple dibloc ou tribloc, comprenant lesdits composés monomériques à effet optique selon l'invention et des comonomères additionnels. Les composés monomériques selon l'invention peuvent former tout ou partie d'un bloc, ou séquence, voire de plusieurs blocs ou séquences. On peut ainsi préparer des copolymères séquencés du type A-B, ABA, BAB, ABC où A est une séquence comprenant le ou les composés monomériques selon l'invention, éventuellement en mélange avec des comonomères additionnels, B et C étant des séquences distinctes, comprenant des comonomères additionnels, seuls ou en mélange, et identiques ou différents des comonomères présents dans la séquence A.
Les copolymères comprenant les composés monomériques (I) peuvent également être du type gradient.

Dans ces copolymères, les composés monomériques à effet optique peuvent être présents en une quantité de 0,01 à 70% en poids par rapport au poids du polymère final, notamment en une quantité de 0,1% à 50% en poids, en particulier de 0,5 à 30% en poids, voire de 1 à 20% en poids, encore mieux de 2 à 10% en poids, les comonomères additionnels, seuls ou en mélange, représentant le complément à 100% en poids.

Les copolymères peuvent comprendre, en plus du ou des composés monomériques à effet optique, au moins un comonomère additionnel qui est hydrophile, ou un mélange de tels comonomères.
Ces comonomères hydrophiles peuvent être présents à raison de 1 à 99,99 % en poids, notamment 2-70% en poids, encore mieux 5-50% en poids, voire 10-30% en poids, par rapport au poids total du copolymère.

Dans la présente description, on désignera indifféremment par 'monomère hydrophile' les monomères dont les homopolymères sont solubles ou dispersibles dans l'eau, ou dont une forme ionique l'est.
Un homopolymère est dit hydrosoluble s'il forme une solution limpide lorsqu'il est en solution à 5% en poids dans l'eau, à 25°C.
Un homopolymère est dit hydrodispersible si, à 5% en poids dans l'eau, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.
Un monomère sera dit 'hydrophobe' s'il n'est pas hydrophile.

De préférence, le ou les, comonomère additionnel hydrophile a une Tg supérieure ou égale à 20°C, notamment supérieure ou égale à 50°C, mais peut éventuellement avoir une Tg inférieur ou égale à 20°C.

Les copolymères selon l'invention peuvent comprendre au moins un comonomère additionnel hydrophobe, ou un mélange de tels comonomères.
Ces comonomères additionnels hydrophobes peuvent être présents à raison de 1 à 99,99% en poids, notamment 30-98% en poids, encore mieux 50-95% en poids, voire 70-90% en poids, par rapport au poids total du copolymère.
De préférence, le comonomère hydrophobe a une Tg supérieure ou égale à 20°C, notamment supérieure ou égale à 30°C, mais peut éventuellement avoir une Tg inférieur ou égale à 20°C.

Dans la présente invention, la Tg (ou température de transition vitreuse) est mesurée selon la norme ASTM D3418-97, par analyse enthalpique différentielle (DSC "Differential Scanning Calorimetry") sur calorimètre, sur une plage de température comprise entre -100°C et +150°C à une vitesse de chauffe de 10°C/min dans des creusets en aluminium de 150 µl.

D'une manière générale, comme comonomère additionnel susceptible d'être copolymérisé avec au moins un composé monomérique de formule (I), on peut citer, seul ou en mélange, les monomères suivants :
- (i) les hydrocarbures éthyléniques ayant 2 à 10 carbones, tels que l'éthylène, l'isoprène, ou le butadiène ;
- (ii) les (méth)acrylates de formule: 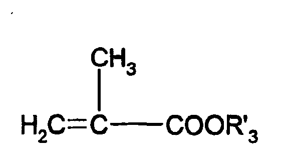 CH₂ = CHCOOR'₃ ou
   dans lesquelles R'₃ représente :
   - un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (CI, Br, I et F), et les groupes Si (R₄R₅), où R₄ et R₅ identiques ou différents représentent un groupe alkyle en C₁ à C₆ ou un groupe phényle ;
      notamment R'3 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle; ou un groupe hydroxyalkyle en C₁₋₄ tel que 2-hydroxyéthyle, 2-hydroxybutyle et 2-hydroxypropyle; ou un groupe alcoxy (C₁₋₄) alkyle (C₁₋₄) tel que méthoxyéthyle, éthoxyéthyle et méthoxypropyle,
   - un groupe cycloalkyle en C₃ à C₁₂, tel que le groupe isobornyle,
   - un groupe aryle en C₃ à C₂₀ tel que le groupe phényle,
   - un groupe aralkyle en C₄ à C₃₀ (groupe alkyle en C₁ à C₈) tel que 2-phényl-éthyle, t-butylbenzyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocycloalkyle (alkyle de 1 à 4 C), tel que furfurylméthyle ou tétrahydrofurfurylméthyle,
      lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle, les atomes d'halogène (CI, Br, I et F), et les groupes Si (R₄R₅), où R₄ et R₅ identiques ou différents représentent un groupe alkyle en C₁ à C₆, ou un groupe phényle,
   - R'₃ peut également être un groupe -(C₂H₄O)ₘ-R", avec m = 5 à 150 et R" = H ou alkyle de C₁ à C₃₀, par exemple -POE-méthyle ou -POE-béhényle;
- (iii) les (méth)acrylamides de formule : 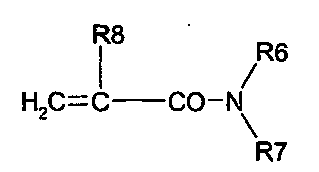 dans laquelle R₈ désigne H ou méthyle; et R₇ et R₆ identiques ou différents représentent :
   - un atome d'hydrogène; ou
   - un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (CI, Br, I et F), et les groupes Si (R₄R₅), où R₄ et R₅ identiques ou différents représentent un groupe alkyle en C₁ à C₆ ou un groupe phényle ;
      notamment R6 et/ou R7 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle; ou un groupe hydroxyalkyle en C₁₋₄ tel que 2-hydroxyéthyle, 2-hydroxybutyle et 2-hydroxypropyle; ou un groupe alcoxy (C₁₋₄) alkyle (C₁₋₄) tel que méthoxyéthyle, éthoxyéthyle et méthoxypropyle,
   - un groupe cycloalkyle en C₃ à C₁₂, tel que le groupe isobornyle,
   - un groupe aryle en C₃ à C₂₀ tel que le groupe phényle,
   - un groupe aralkyle en C₄ à C₃₀ (groupe alkyle en C₁ à C₈) tel que 2-phényl-éthyle, t-butylbenzyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocycloalkyle (alkyle de 1 à 4 C), tel que furfurylméthyle ou tétrahydrofurfurylméthyle,
      lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle, les atomes d'halogène (CI, Br, I et F), et les groupes Si (R₄R₅), où R₄ et R₅ identiques ou différents représentent un groupe alkyle en C₁ à C₆, ou un groupe phényle. Des exemples de monomères (méth)acrylamide sont le (méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylacrylamide, l'undécylacrylamide, et le N(2-hydroxypropylméthacrylamide).
- (iv) les composés vinyliques de formules :

   CH₂=CH-R₉, CH₂=CH-CH₂-R₉ ou CH₂=C(CH₃)-CH₂-R₉

   dans lesquelles R₉ est un groupe hydroxyle, halogène (Cl ou F), NH₂, OR₁₄ où R₁₄ représente un groupe phényle ou un groupe alkyle en C₁ à C₁₂ (le monomère est un éther de vinyle ou d'allyle); acétamide (NHCOCH₃); un groupe OCOR₁₅ où R₁₅ représente un groupe alkyle de 2 à 12 carbones, linéaire ou ramifié (le monomère est un ester de vinyle ou d'allyle) ; ou un groupe choisi parmi :
   - un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (CI, Br, I et F), et les groupes Si (R₄R₅), où R₄ et R₅ identiques ou différents représentent un groupe alkyle en C₁ à C₆ ou un groupe phényle ;
   - un groupe cycloalkyle en C₃ à C₁₂ tel que isobornyle, cyclohexane,
   - un groupe aryle en C₃ à C₂₀ tel que phényle,
   - un groupe aralkyle en C₄ à C₃₀ (groupe alkyle en C₁ à C₈) tel que 2-phényléthyle ; benzyle,
   - un groupe hétérocyclique de 4 à 12 chaînons contenant un ou plusieurs hétéroatomes choisis parmi O, N, et S, le cycle étant aromatique ou non,
   - un groupe hétérocycloalkyle (alkyle de 1 à 4 C), tel que furfurylméthyle ou tétrahydrofurfurylméthyle,
   lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène (CI, Br, I et F) et les groupes Si(R₄R₅) où R₄ et R₅ identiques ou différents représentent un groupe alkyle en C₁ à C₆, ou un groupe phényle.
   Des exemples de monomères vinyliques sont le vinylcyclohexane, et le styrène. Des exemples d'esters de vinyle sont l'acétate de vinyle le propionate de vinyle, le butyrate de vinyle, l'éthylhexanoate de vinyle, le néononanoate de vinyle et le néododécanoate de vinyle.
   Parmi les éthers de vinyle, on peut citer le vinyl méthyl éther, le vinyl éthyl éther et le vinyl isobutyl éther.
- (v) les monomères (méth)acryliques, (méth)acrylamides ou vinyliques à groupe fluoré ou perfluoré, tels que le (méth)acrylate d'éthyl-perfluorooctyle ou d'éthyl-2-perfluorohexyle;
- (vi) les monomères (méth)acryliques, (méth)acrylamides ou vinyliques siliconés, tels que le méthacryloxypropyltris(triméthylsiloxy)silane ou l'acryloxypropylpoly-diméthylsiloxane.
- (vii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- (viii) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

Les sels peuvent être formés par neutralisation des groupes anioniques à l'aide d'une base minérale, telle que LiOH, NaOH, KOH, Ca(OH)₂, NH₄OH ou Zn(OH)₂; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(dimethylamino)-propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

On peut bien évidemment utiliser plusieurs des comonomères additionnels ci-dessus mentionnés.

Le ou les comonomères additionnels peuvent être présents en une quantité de 30% à 99,99% en poids par rapport au poids du polymère final, notamment en une quantité de 50% à 99,9% en poids, en particulier de 70% à 99,5% en poids, voire de 80 à 99% en poids, encore mieux de 90 à 98% en poids.

On choisit plus particulièrement les comonomères additionnels parmi, seuls ou en mélange, les (méth)acrylates d'alkyle en C1-C18 ou de cycloalkyle en C3-C12, et notamment parmi l'acrylate de méthyle, le méthacrylate de méthyle, l'acrylate d'isobornyle, le méthacrylate d'isobornyle, l'acrylate d'isobutyle, le méthacrylate d'isobutyle, l'acrylate d'éthyl-2-hexyle, le méthacrylate d'éthyl-2-hexyle, l'acrylate de dodécyle, le méthacrylate de dodécyle, l'acrylate de stéaryle, le méthacrylate de stéaryle, l'acrylate de trifluoroéthyle, le méthacrylate de trifluoroéthyle.
On peut également citer l'acide acrylique, l'acide méthacrylique, le méthacryloxypropyltris(triméthylsiloxy)silane, l'acryloxypropyltris(triméthylsiloxy)silane, l'acryloxypropylpolydiméthylsiloxane et le méthacryloxypropylpolydiméthylsiloxane.

Lesdits polymères peuvent être préparés selon les méthodes connues de l'homme du métier, notamment par polymérisation radicalaire; polymérisation radicalaire contrôlée, par exemple par les xanthanes, les dithiocarbamates ou les dithioesters; par polymérisation à l'aide de précurseurs de type nitroxydes; par polymérisation radicalaire par transfert d'atomes (ATRP); par polymérisation par transfert de groupe.
D'une manière classique, la polymérisation peut être effectuée en présence d'un initiateur de polymérisation, qui peut être un amorceur radicalaire, et notamment qui peut être choisi parmi les composés organiques peroxydés tels que le dilauroyl peroxyde, le dibenzoyl peroxyde, le ter-butyl peroxy-2-éthylhexanoate; ou bien parmi les composés diazotés tels que l'azobisisobutyronitrile ou l'azobisdiméthylvaléronitrile. La réaction peut également être initiée à l'aide de photoinitiateurs ou par une radiation de type UV, par des neutrons ou par plasma.

Les composés monomériques à effet optique, ainsi que de préférence les homoou co-polymères les comprenant, présentent de préférence une longueur d'onde d'absorption comprise entre 200 et 550 nm, notamment entre 220 et 520 nm, voire entre 240 et 500 nm.
Ils présentent de préférence une longueur d'onde d'émission comprise entre 350 et 750 nm, notamment entre 390 et 700 nm, voire entre 420 et 670 nm.

La masse moléculaire moyenne en poids (Mw) des copolymères utilisés selon l'invention est de préférence comprise entre 5000 et 600 000 g/mol, notamment entre 10 000 et 300 000 g/mol, et encore mieux entre 20 000 et 150 000 g/mol.
On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique et UV.

Les polymères, qu'ils soient homopolymères ou copolymères, peuvent être présents, seuls ou en mélange, dans les compositions selon l'invention en une quantité de 0,01 à 60% en poids, de préférence 0,1 à 50% en poids, notamment 1 à 25% en poids, voire 3 à 15% en poids, et encore mieux 5 à 12% en poids, par rapport au poids total de la composition.

Ils peuvent être présents dans la composition sous forme solubilisée, par exemple dans l'eau, dans une huile ou dans un solvant organique, ou bien sous forme de dispersion aqueuse ou organique.
Avantageusement, les polymères utilisés selon l'invention sont solubles ou dispersibles dans au moins une des phases de la composition qui les comprend.

Les compositions cosmétiques ou pharmaceutiques selon l'invention comprennent, outre lesdits polymères, un milieu physiologiquement acceptable, notamment cosmétiquement, dermatologiquement ou pharmaceutiquement, acceptable, c'est-à-dire un milieu compatible avec les matières kératiniques telles que la peau du visage ou du corps, les cheveux, les cils, les sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en C₂ et des aldéhydes en C₂-C₄ hydrophiles.

L'eau ou le mélange d'eau et de solvants organiques hydrophiles peut être présent dans la composition selon l'invention en une teneur allant de 0,1% à 99% en poids, par rapport au poids total de la composition, et de préférence de 10% à 80% en poids.
La composition peut également être anhydre.

La composition peut également comprendre une phase grasse qui peut comprendre des corps gras liquides à température ambiante (25°C en général) et/ou des corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.
Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.
Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.
Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.
On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.
Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon invention peut en outre comprendre, dans une phase particulaire, des pigments et/ou des nacres et/ou des charges habituellement utilisés dans les compositions cosmétiques.
La composition peut également comprendre d'autres matières colorantes choisies parmi les colorants hydrosolubles et/ou les colorants liposolubles bien connus de l'homme du métier.
Par pigments, il faut comprendre des particules de toute forme, blanches ou colorées, minérales ou organiques, insolubles dans le milieu physiologique, destinées à colorer la composition.
Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Par nacres, il faut comprendre des particules de toute forme irisées, notamment produites par certains mollusques dans leur coquille ou bien synthétisées.
Les pigments peuvent être présents dans la composition à raison de 0,01 à 25% en poids de la composition finale, et de préférence à raison de 3 à 10% en poids. Ils peuvent être blancs ou colorés, minéraux ou organiques,. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.
Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.

La composition selon l'invention peut comprendre en outre en outre une ou plusieurs charges, notamment en une teneur allant de 0,01% à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Coming) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre. Cette composition peut avoir l'aspect d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

Elle peut ainsi se présenter sous la forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cemes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).
Elle peut également se présenter sous forme d'une composition de protection ou de soin de la peau du visage, du cou, des mains ou du corps, notamment une composition anti-rides, une composition hydratante ou traitante; une composition anti-solaire ou de bronzage artificiel.
Elle peut encore se présenter sous forme d'un produit capillaire, notamment pour la coloration, le maintien de la coiffure, la mise en forme des cheveux, le soin, le traitement ou le nettoyage des cheveux, telle que des shampooings, des gels, des lotions de mise en plis, des lotions pour le brushing, des compositions de fixation et de coiffage telles que les laques ou spray.

L'invention a aussi pour objet un procédé cosmétique de maquillage ou de soin des matières kératiniques, notamment de la peau du corps ou du visage, des lèvres, des ongles, des cils, des sourcils et/ou des cheveux, comprenant l'application sur lesdites matières d'une composition cosmétique telle que définie précédemment.

L'invention est illustrée plus en détail dans les exemples suivants.

### Méthode de mesure de la longueur d'ondes (émission et absorption)

La mesure des longueurs d'ondes est réalisée à l'aide d'un fluorimètre Varian Cary Eclipse.
Sauf indication contraire, cette mesure est effectuée de la manière suivante : On dispose 20 mg de produit dans un cylindre de 50 ml. Afin de solubiliser le produit, on complète ledit cylindre jusqu'à 50 ml, à l'aide d'un solvant approprié, par exemple le dichlorométhane (DCM), le chloroforme ou le diméthylsulfoxyde (DMSO). La solution résultante est mélangée et on en prélève 250 microlitres que l'on dispose dans un cylindre de 50 ml, puis que l'on complète à nouveau avec le solvant jusqu'à 50 ml.
On mélange le tout et l'on prélève un échantillon de la solution que l'on dispose dans une cuve fermée, en quartz et d'épaisseur 10 mm, qui est alors placée dans la chambre de mesure.

### Exemple 1

### 1/ première étape

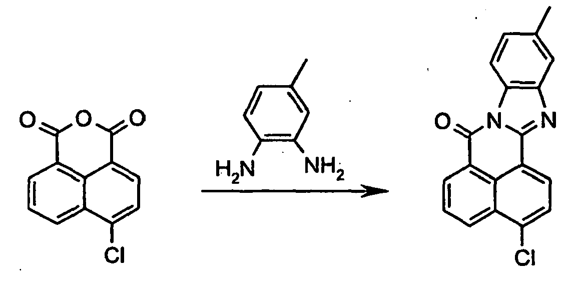

Dans un tricol de 1 litre, sous atmosphère inerte (azote), on dispose 179,9 g (0,77 mol) de 4-chloro-1,8-naphthalic anhydride, puis l'on ajoute 1 litre de 2-(2-éthoxyéthoxy)-éthanol. Le mélange est chauffé à 100°C et il devient limpide orange. Sous agitation, on introduit goutte-à-goutte 100 g (0,78 mol) de 3,4-diaminotoluène préalablement mis en solution dans 300 ml de 2-(2-éthoxyéthoxy)-éthanol. On rince l'ampoule avec 200 ml de ce solvant. On chauffe la solution réactionnelle à 150°C pendant 18 heures. Le mélange réactionnel résultant est alors laissé à refroidir à température ambiante. Le précipité est filtré sur fritté, puis lavé à l'éthanol. On le récupère et sèche sous vide. On obtient 211,3 g de cristaux orange (rendement 72,6%).

### Caractérisation

¹H-NMR (CDCl₃, 400MHz) δ : 8.74 - 8.72 (1H), 8.61-8.42 (3H), 7.83 - 7.56 (3H), 7.24 - 7.22 (1 H), 2.54 - 2.51 (3H).

### 2/ deuxième étape

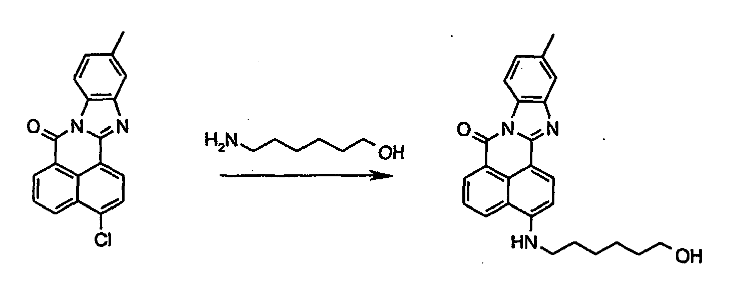

On dispose 13,6 g (42,7 mmol) de *p*-tolyl-4-chloroisoquinolinone dans un ballon tricol équipé d'un condensateur Dimroth et placé sous atmosphère inerte d'argon, et l'on ajoute 100,0 g (0,85 mol) de 6-aminohexane-1-ol. On mélange et l'on chauffe à 180°C afin d'obtenir un mélange homogène; on laisse réagir pendant 16 heures puis on laisse refroidir jusqu'à température ambiante et un précipité rouge se forme. On filtre ledit précipité et on le lave à l'éthanol. On obtient 17,0 g de produit recherché.

### Caractérisation

¹H-NMR (DMSO, 400MHz) δ : 8.82-8.20 (4H), 7.96-7.49 (3H), 7.28-7.15 (1H), 6.82-6.80 (1 H), 4.37 (1 H), 3.41-3.39 (2H), 2.49-2.45 (5H), 1.72-1.69 (2H), 1.48-1.36 (6H).

### 3/ Troisième étape

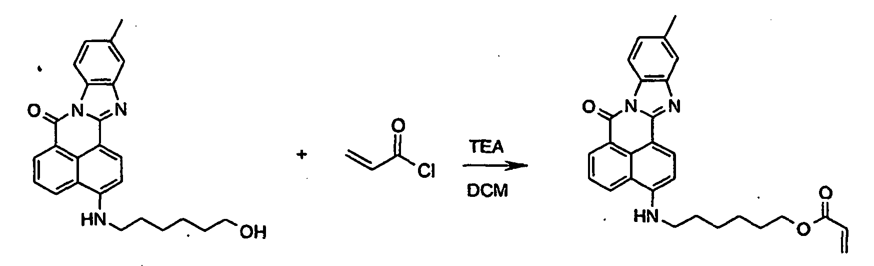

On dispose 5,0 g (12,5 mmol) de *p*-tolyl-4-(hexan-6-ol)-isoquinolinone dans un ballon tricol équipé d'un condensateur Dimroth et placé sous atmosphère inerte d'argon. On ajoute 150 ml de dichlorométhane sec et l'on mélange jusqu'à obtention d'une solution homogène. On ajoute alors 7,0 ml (50 mmol) de triéthanolamine puis 4,1 ml (50 mmol) de chlorure d'acryloyle, sous agitation à 0°C. On laisse la température augmenter jusqu'à 25°C. On suit l'évolution de la réaction par CCM et lorsque l'on constate que tout a réagi, on ajoute 50 ml d'eau. La solution réactionnelle est alors lavée avec de l'eau salée + bicarbonate de sodium (brine), puis encore de l'eau, et séchée sur sulfate de sodium. On évapore les solvants sous pression réduite et l'on obtient 4,1g (rendement 73,5%) d'une poudre rouge.

### Caractérisation

¹H-NMR (DMSO, 500MHz) δ: 8.70 - 8.68 (1H), 8.63 - 8.60 (1H), 8.44 - 8.42 (1H), 8.33 - 8.29 (1 H), 7.90 - 7.88 (1 H), 7.73 - 7.61 (2H), 7.27 - 7.23 (1 H), 6.82 - 6.81 (1H), 6.32 - 6.29 (1H), 6.18 - 6.12 (1 H), 5.92 - 5.89 (1H), 4.13 - 4.11 (2H), 3.41 - 3.37 (2H), 1.74 - 1.62 (4H), 1.47 - 1.40 (4H).
λₘₐₓabsorption : 377 nm
λₘₐₓémission : 556 nm (rouge)

### Exemple 2

### 1/ première étape

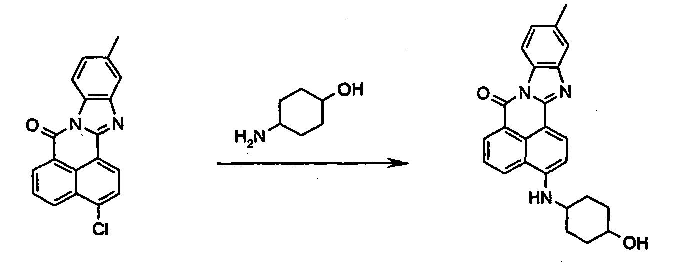

On dispose 8,0 g (25,1 mmol) de *p*-tolyl-4-chloro-isoquinolinone et 8,7g de transaminocyclohexanol (75,3 mmol, 3eq) dans un réacteur pour microonde: On ajoute 30 ml de NMP. Le réacteur est placé dans la cuve du micro onde avec une pale d'agitation en verre. On chauffe sous agitation jusqu'à 130°C en 5 minutes puis on maintient à 130°C pendant 4 heures. Le milieu solide devient petit à petit liquide et passe d'une solution pâteuse vert jaune à une solution liquide rouge bordeaux. On réalise une CCM (CH₂Cl₂ : MeOH, 9 : 1) pour constater la fin de la réaction. On verse alors la solution réactionnelle dans 600 ml d'une solution d'hydrogénocarbonate de sodium; le produit précipite aussitôt; on le lave trois fois avec 600 ml d'eau, puis on le sèche sous vide et l'on obtient 9,07 g de poudre rouge bordeaux brillante (rendement 90,9%).

### Caractérisation

¹H-NMR (DMSO, 400MHz) δ: 8.89 - 8.65 (2H), 8.50 - 8.42 (1 H), 8.34 - 8.23 (1 H), 7.76 - 7.18 (4H), 6.94 - 6.91 (1H), 4.65 (1 H), 3.66 - 3.63 (1 H), 3.52 - 3.47 (1 H), 2.51 - 2.47 (3H), 2.09 - 2.02 (2 H), 1.94 -1.92 (2H), 1.55 - 1.37 (4H).

### 2/ deuxième étape

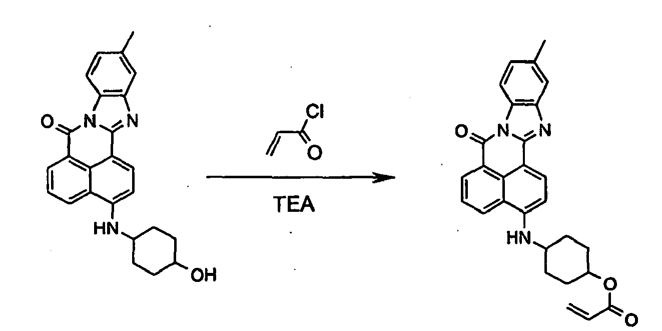

Dans un tricol de 1 litres, sous atmosphère inerte (argon), on dispose 10,0 g (25 mmol) de *p*-tolyl-4-(cyclohexylamino-4-hydroxy)-isoquinolinone puis l'on ajoute 350 ml de dichlorométhane. On mélange jusqu'à obtention d'une solution homogène. On introduit alors 3,31 g (33 mmol) de triéthanolamine. Sous agitation (500 tr/min) à 5°C, on introduit goutte-à-goutte 3,0 g (33 mmol) de chlorure d'acryloyle dans 50 ml de dichlorométhane, puis on chauffe la solution réactionnelle à 40°C. La réaction est suivie en CCM jusqu'à disparition du produit de départ. Après 3 heures de réaction, on lave la phase organique à l'eau et au bicarbonate de sodium, et encore avec l'eau. On sèche la phase organique sur sulfate de sodium, et on évapore les solvants. On obtient le produit brut qui est purifié sur silice. On récupère 5,1 g du produit recherché (rendement 45.2%).

### Caractérisation

¹H-NMR (DMSO, 400MHz) δ: 8.89-8.65 (2H), 8.50-8.42 (1 H), 8.34-8.23 (1 H), 7.76-7.18 (4H), 6.94-6.91 (1H), 6.37-6.32 (1H), 6.23-6.16 (1H), 5.96-5.94 (1H), 4.79-4.76 (1H), 3.75-3.74 (1H), 2.49-2.45 (3H), 2.11-2.05 (4 H), 1.68-1.58 (4H).
λₘₐₓabsorption : 472 nm
λₘₐₓémission : 587 nm

### Exemple 3

On prépare un homopolymère selon l'invention à partir du monomère de l'exemple 1.

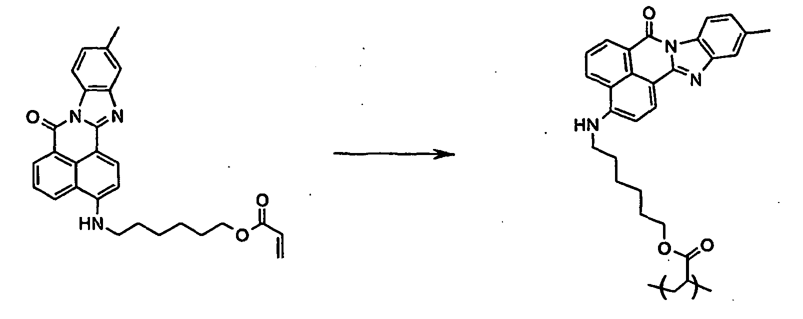

On dissout 1,0 g (2,2 mmol) de monomère préparé à l'exemple 1 dans 15 ml de THF anhydre, à 60°C, en présence de 180 µl d'initiateur de polymérisation (Trigonox 21S). On chauffe jusqu'à 90°C sous agitation, puis on maintient l'agitation pendant 20 heures. La viscosité de la solution augmente; on dilue le milieu par ajout de 20 ml de THF, puis on le précipite goutte à goutte dans 500 ml d'acétone refroidi à 0°C. On sèche le polymère obtenu à l'étuve (50°C) sous pression réduite.

On obtient 0,56 g d'homopolymère (rendement : 56 %).
λₘₐₓabsorption : 578 nm
λₘₐₓémission : 620 nm

### Exemple 4

On prépare un homopolymère selon l'invention à partir du monomère de l'exemple 2.

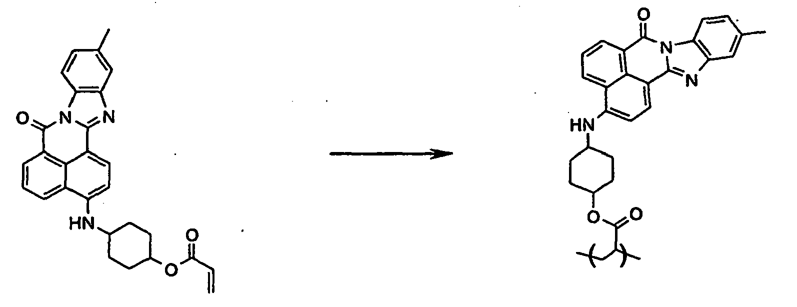

On dissout 1,0 g (2,2 mmol) de monomère préparé à l'exemple 2 dans 15 ml de THF anhydre, à 60°C, en présence de 180 µl d'initiateur de polymérisation (Trigonox 21 S). On chauffe jusqu'à 90°C sous agitation, puis on maintient l'agitation pendant 18 heures. La viscosité de la solution augmente; on dilue le milieu par ajout de 20 ml de THF, puis on le précipite goutte à goutte dans 500 ml d'acétone refroidi à 0°C. On sèche le polymère obtenu à l'étuve (50°C) sous pression réduite.

On obtient 0,54 g d'homopolymère (rendement : 54%).
λₘₐₓabsorption : 472 nm
λₘₐₓémission : 587 nm

### Exemple 5

On prépare un copolymère statistique selon l'invention.

On dissout 4 g du monomère préparé à l'exemple 1 dans 20 g de DMSO puis on ajoute de l'acrylate d'isobomyle (33 g), du méthacrylate d'isobomyle (33 g), de l'acrylate d'isobutyle (30 g), de l'isododécane (80 g) et 1 g d'amorceur 2,5-bis(2-éthylhexanoylperoxy)-2,5-diméthylhexane (Trigonox^{®}141 d'Akzo Nobel). On chauffe jusqu'à 120°C et l'on maintient le mélange à cette température pendant 4 heures. On laisse ensuite le mélange refroidir à température ambiante, et, après remplacement du DMSO par de l'isododécane, on obtient une solution à 50% de matière sèche de polymère dans l'isododécane.
Ce polymère présente une masse moyenne en poids de 43600 et un indice de polydispersité Ip de 6,8.

### Exemple 6

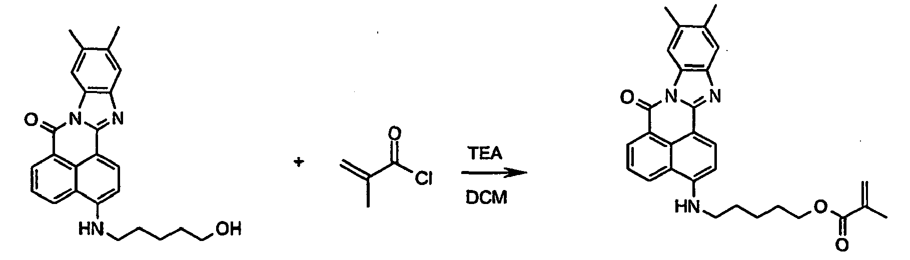

On dispose 4,0 g (10,01 mmol) de diméthylbenzoisoquinolinoneaminopentanol dans un ballon tricol équipé d'un condensateur Dimroth et placé sous atmosphère inerte d'argon. On ajoute 200 ml de dichlorométhane sec (DCM) et l'on mélange jusqu'à obtention d'une solution homogène. On ajoute alors 3 g (30 mmol) de triéthanolamine puis 1,9 g (18,0 mmol) de chlorure de méthacryloyle dilué dans 15 ml de DCM, sous agitation à 45°C. On suit l'évolution de la réaction par CCM. Lorsque l'on constate que tout a réagi (environ 24 heures), on lave la solution réactionnelle avec de l'eau salée + bicarbonate de sodium (brine), puis encore de l'eau, et on la sèche sur sulfate de sodium. On évapore les solvants sous pression réduite et l'on obtient 1,64 g (rendement 35%) d'une poudre de couleur rouge-orangée.

### Caractérisation

¹H-NMR (CDCl₃, 400MHz) δ : 8.63 - 8.60 (1H), 8.40-8.25 (2H), 7.82 - 7.79 (1H), 7.58 - 7.47 (2H), 6.53 - 6.50 (1H), 6.11 (1H), 5.57-5.56 (1H), 5.23 - 5.20 (1H), 4.21- 4.17 (2H), 3.20 - 3.15 (2H), 2.44-2.37 (6H), 1.95 (3H), 1.76-1.66 (4H), 1.53 - 1.45 (2H).

### Exemple 7

On prépare un homopolymère selon l'invention à partir du monomère de l'exemple 6.

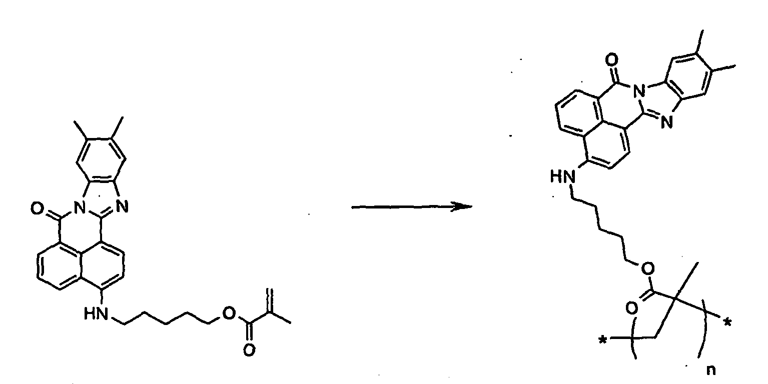

On dissout 0,5 g (1,07 mmol) de monomère préparé à l'exemple 6 dans 25 ml de THF anhydre, à 90°C, en présence de 0,05 g d'initiateur de polymérisation (Trigonox 141S). On chauffe jusqu'à 90°C sous agitation, puis on maintient pendant 30 heures. On précipite le polymère goutte à goutte dans 500 ml d'acétone refroidi à - 10°C. On sèche le polymère obtenu à l'étuve (60°C) sous pression réduite.
On obtient 0,13 g d'homopolymère, sous forme de poudre de couleur orange.

### Exemple 8

On prépare un copolymère statistique selon l'invention.

On dissout 6 g du monomère préparé à l'exemple 1 dans 60 ml de THF, à 60°C, puis on ajoute 14 g d'acrylate d'éthyl 2-hexyle et 10 ml d'isododécane. On chauffe à 80°C, sous agitation pendant 30 minutes, puis on ajoute 0,4 g d'initiateur (Trigonox 21S) dilué dans 10 ml de THF et 5 ml d'isododécane. On chauffe jusqu'à 90°C et l'on maintient le mélange à cette température pendant 24 heures. On laisse ensuite le mélange refroidir à température ambiante, et, après remplacement du THF par de l'isododécane, on obtient une solution à 50% de matière sèche de polymère dans l'isododécane.

### Exemple 9

On prépare un fond de teint anhydre comprenant (% en poids) :
- cire de polyéthylène 12%
- huiles siliconées volatiles 25%
- phényltriméthicone 20%
- Microsphères de polyméthylméthacrylate 12%
- Polymère de l'exemple 5 6% MA
- Isododécane qsp 100%
(MA : matière active)

Préparation : On fait fondre les cires puis, quand tout est limpide, on ajoute la phényl triméthicone sous agitation, et les huiles de silicones; on ajoute ensuite les microsphères, l'isododécane et le polymère. On homogénéise pendant 15 minutes puis on coule la composition résultante que l'on laisse refroidir. On obtient un fond de teint anhydre.

### Exemple 10

On prépare un copolymère statistique comprenant un monomère selon l'invention.

Dans un réacteur, sous argon, équipé d'un condensateur et d'une agitation, on introduit 20 g d'isododécane, puis 27 g de méthacrylate de méthyle, 17 g d'acrylate de méthyle et 5 g d'acide acrylique. On mélange et l'on ajoute un mélange constitué de 1 g de monomère de l'exemple 2 dans 20,0 g de toluène.
On ajoute 0,5 g de Trigonox 21 S (peroxy-2-éthylhexanoate de t-butyle) puis on chauffe le mélange réactionnel à 90°C; on maintient l'agitation et le chauffage pendant 6 heures puis on refroidit à température ambiante. Le polymère résultant est purifié par précipitation.
On obtient un polymère statistique comprenant (% en poids) : 54% de méthacrylate de méthyle, 34% acrylate de méthyle, 10% d'acide acrylique et 2% de monomère selon l'invention.

### Exemple 11

On prépare un vernis à ongles comprenant :
- 5% en poids de polymère selon l'exemple 7
- qsp 100% solvants organiques.

## Revendications

1. Composition cosmétique ou pharmaceutique comprenant, dans un milieu physiologiquement acceptable, au moins un polymère comprenant au moins un composé monomérique de formule (I) : 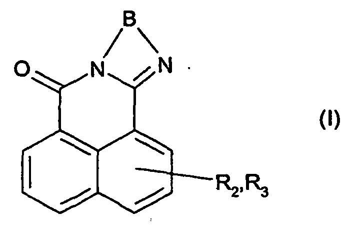 dans laquelle :
- R2 et R3, présents sur le même cycle ou chacun sur un cycle différent, représentent, indépendamment l'un de l'autre, un hydrogène, un halogène, ou un groupement de formule -X-G-P (II), sous réserve qu'au moins l'un des radicaux R2 et/ou R3 représente un groupement de formule (II), dans laquelle :
- X est choisi parmi les groupements -O-, -S-, -SO-, -SO₂-, -NH- ou -NR- avec R représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- G est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- P est un groupement polymérisable choisi parmi l'une des formules suivantes : 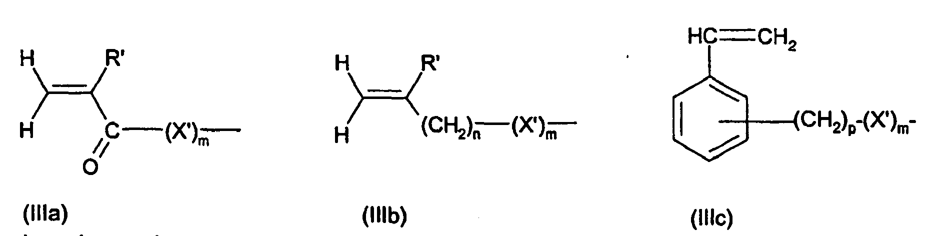 dans lesquelles:
- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyles et/ou aryles pouvant en outre être substitués par un ou plusieurs groupements choisis parmi OH, halogène, alcoxy en C1-6 et aryloxy en C6-10; de préférence X' représente O;
- m est égal à 0 ou 1; n est égal à 0 ou 1; p est égal à 0, 1 ou 2.
- B représente l'un des groupements aromatiques divalents suivants (IVa) à (IVd) : 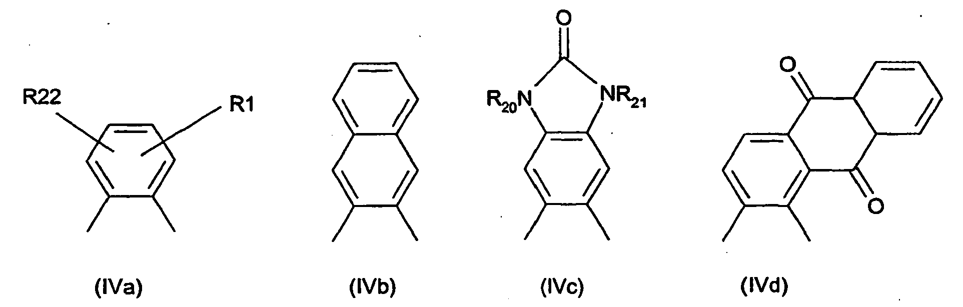 dans lesquels :
- R1 est un radical carboné linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S.
- R22 est un atome d'hydrogène ou un radical carboné linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- R20 et R21 sont, indépendamment l'un de l'autre, un atome d'hydrogène, un radical alkyle linéaire ou ramifié en C1-8, un radical cyclopentyle, cyclohexyle, cyclooctyle, cyclodécyle, cyclododécyle, benzyle, naphtyle ou phényle.

2. Composition selon l'une des revendications précédentes, dans laquelle, dans le composé monomérique, le radical R2 est un atome d'hydrogène et R3 un groupement de formule (II).

3. Composition selon l'une des revendications précédentes, dans laquelle, dans le composé monomérique, dans le groupement de formule (II), X est choisi parmi -O-, -NH- et -NR- avec R représentant préférentiellement un radical hydrocarboné linéaire, ramifié et/ou cyclique, saturé ou insaturé, comprenant éventuellement un cycle hydrocarboné lui-même saturé ou insaturé, comprenant 2 à 18, notamment 3 à 12 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S.

4. Composition selon l'une des revendications précédentes, dans laquelle, dans le composé monomérique, X est choisi parmi -NH- et -NR- avec R représentant un cyclohexyle.

5. Composition selon l'une des revendications précédentes, dans laquelle, dans le composé monomérique, le radical divalent G est un radical hydrocarboné divalent linéaire, ramifié et/ou cyclique, saturé ou insaturé, comprenant éventuellement un cycle hydrocarboné lui-même saturé ou insaturé, comprenant au total 2 à 18, notamment 3 à 10 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si.

6. Composition selon l'une des revendications précédentes, dans laquelle, dans le composé monomérique, G est choisi parmi les radicaux hydrocarbonés divalents linéaires ou ramifiés, saturés comprenant éventuellement un cycle hydrocarboné saturé, comprenant au total 2 à 18, notamment 3 à 10 atomes de carbone.

7. Composition selon l'une des revendications précédentes, dans laquelle, dans le composé monomérique, le groupement polymérisable P est choisi parmi l'une des formules suivantes : 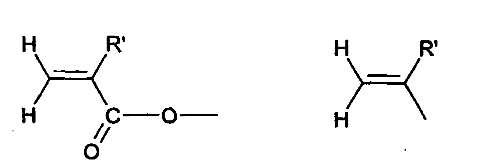 dans lesquelles R' représente H ou méthyle.

8. Composition selon l'une des revendications précédentes, dans laquelle, dans le composé monomérique, le groupement B est choisi parmi ceux de formule (IVa) dans laquelle R1 est préférentiellement un radical carboné linéaire, ramifié et/ou cyclique, saturé, comprenant 1 à 32 atomes de carbone, notamment 2 à 12, voire 3 à 6 atomes de carbone; en particulier R1 peut être un radical méthyle, éthyle ou propyle.

9. Composition selon l'une des revendications précédentes, dans laquelle le composé monomérique répond à l'une des formules suivantes dans lesquelles R est l'hydrogène ou méthyle:

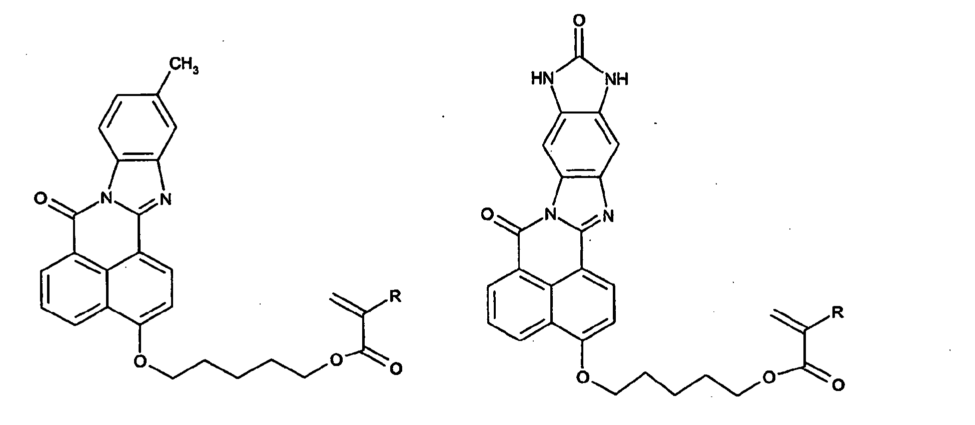

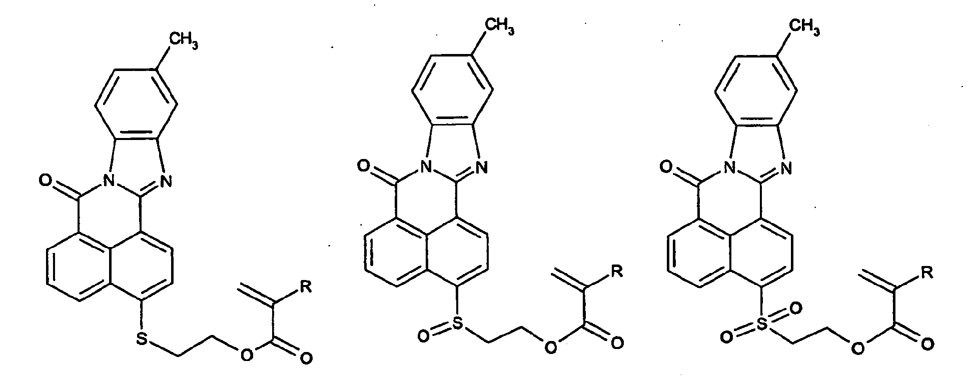

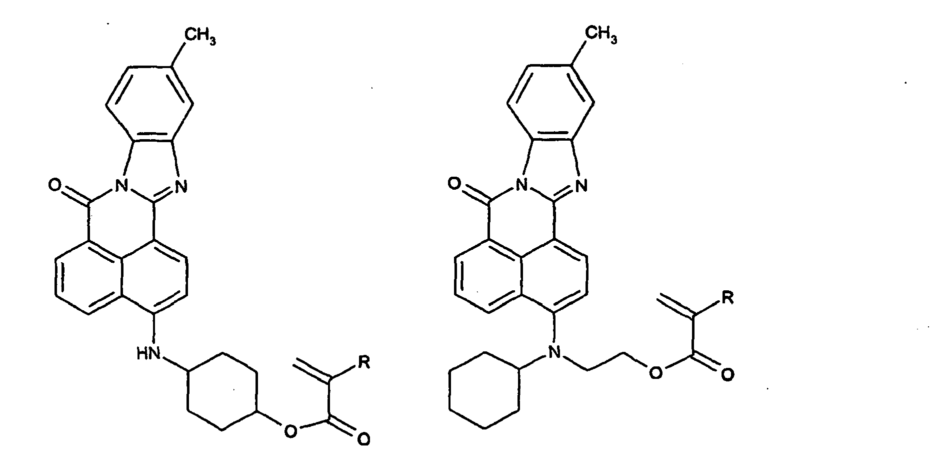

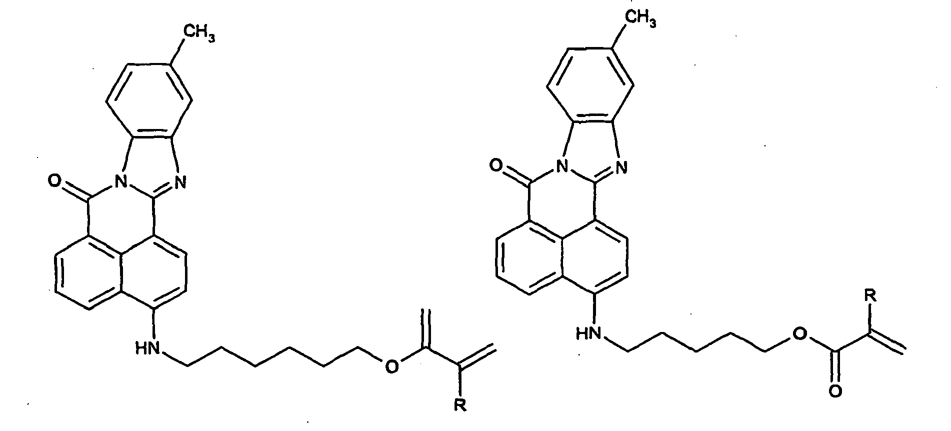

10. Composition selon l'une des revendications précédentes, dans laquelle le polymère est un homopolymère d'un composé monomérique tel que défini à l'une des revendications 1 à 9.

11. Composition selon l'une des revendications 1 à 9, dans laquelle le polymère est un copolymère ne comprenant que des composés monomériques tels que définis à l'une des revendications 1 à 9.

12. Composition selon l'une des revendications 1 à 9, dans laquelle le polymère est un copolymère comprenant au moins un composé monomérique tel que défini à l'une des revendications 1 à 9, et au moins un comonomère additionnel.

13. Composition selon l'une des revendications 11 à 12, dans laquelle le polymère est un copolymère statistique, alterné, greffé, séquencé ou gradient.

14. Composition selon l'une des revendications 11 à 12, dans laquelle le composé monomérique est présent en une quantité de 0,01 à 70% en poids par rapport au poids dudit polymère, notamment en une quantité de 0,1% à 50% en poids, en particulier de 0,5 à 30% en poids, voire de 1 à 20% en poids, encore mieux de 2 à 10% en poids, les comonomères additionnels, seuls ou en mélange, représentant le complément à 100% en poids.

15. Composition selon l'une des revendications 12 à 14, dans laquelle le polymère comprend au moins un comonomère additionnel à effet optique choisi parmi les composés de formule (A) et/ou de formule (B) ci-après : 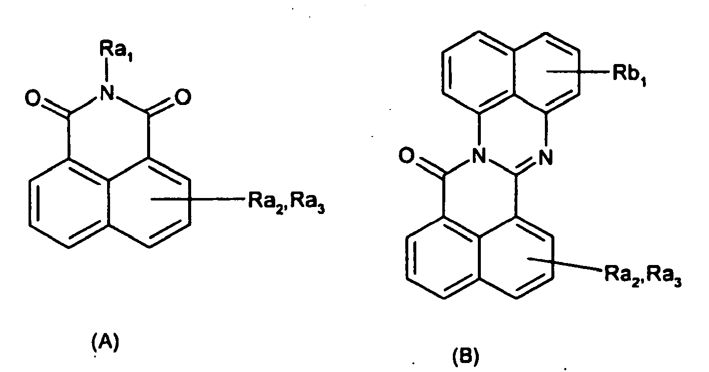 dans laquelle :
- Ra1 représente un radical carboné linéaire, ramifié et/ou cyclique, saturé et/ou insaturé, comprenant 1 à 32 atomes de carbone; éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- Rb1 est choisi parmi (i) un atome d'hydrogène, (ii) un halogène, (iii) un radical carboné linéaire, ramifié et/ou cyclique, saturé et/ou insaturé, comprenant 1 à 12 atomes de carbone, éventuellement substitué par un ou plusieurs groupements choisis parmi =O, OH, NH₂ et/ou éventuellement interrompu par un ou plusieurs hétéroatomes choisis parmi O, N, P, Si et S; (iv) un groupement NRR' avec R et R' étant, indépendamment 'un de l'autre, un atome d'hydrogène ou un radical hydrocarboné, linéaire, cyclique ou ramifié, saturé en C1-6, notamment méthyle, éthyle, propyle, isopropyle, n-butyle, iso-butyle, tert-butyle, pentyle ou hexyle;
- Ra2 et Ra3, présents sur le même cycle ou chacun sur un cycle différent, représentent, indépendamment l'un de l'autre, un hydrogène, un halogène, ou un groupement de formule -Xa-Ga-Pa (II), sous réserve qu'au moins l'un des radicaux Ra2 et/ou Ra3 représente un groupement de formule (II), dans laquelle :
- Xa est choisi parmi les groupements -O-, -S-, -SO-, -SO₂-, -NH- ou -NR4- avec R4 représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- Ga est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, NH₂, et les atomes d'halogènes; et/ou éventuellement interrompu par un ou plusieurs hétéroatomes choisis parmi O, N, P, Si et S;
- Pa est un groupement polymérisable choisi parmi l'une des formules suivantes : 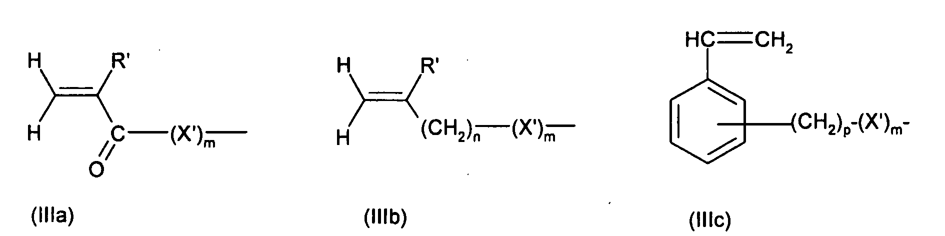 dans lesquelles :
- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyles et/ou aryles pouvant en outre être substitués par un ou plusieurs groupements choisis parmi OH, halogène, alcoxy en C1-6 et aryloxy en C6-10; et
- m est égal à 0 ou 1;n est égal à 0 ou 1; p est égal à 0, 1 ou 2.

16. Composition selon l'une des revendications 12 à 15, dans laquelle le polymère comprend au moins un comonomère additionnel choisi parmi, seul ou en mélange, les monomères suivants :
- (i) les hydrocarbures éthyléniques ayant 2 à 10 carbones, tels que l'éthylène, l'isoprène, ou le butadiène ;
- (ii) les (méth)acrylates de formule: 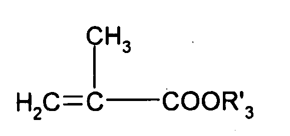
CH₂ = CHCOOR'₃
ou
dans lesquelles R'₃ représente :
- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆ ou un groupe phényle ;
notamment R'3 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle; ou un groupe hydroxyalkyle en C₁₋₄ tel que 2-hydroxyéthyle, 2-hydroxybutyle et 2-hydroxypropyle; ou un groupe alcoxy (C₁₋₄) alkyle (C₁₋₄) tel que méthoxyéthyle, éthoxyéthyle et méthoxypropyle,
- un groupe cycloalkyle en C₃ à C₁₂, tel que le groupe isobornyle,
- un groupe aryle en C₃ à C₂₀ tel que le groupe phényle,
- un groupe aralkyle en C₄ à C₃₀ (groupe alkyle en C₁ à C₈) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,
lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆, ou un groupe phényle,
- R'₃ peut également être un groupe -(C₂H₄O)ₘ-R", avec m = 5 à 150 et R" = H ou alkyle de C₁ à C₃₀, par exemple -POE-méthyle ou -POE-béhényle;
- (iii) les (méth)acrylamides de formule : 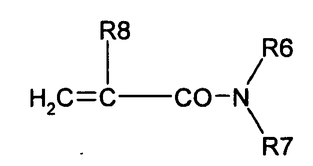 dans laquelle R₈ désigne H ou méthyle; et R₇ et R₆ identiques ou différents représentent :
- un atome d'hydrogène; ou
- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆ ou un groupe phényle ; notamment R6 et/ou R7 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle; ou un groupe hydroxyalkyle en C₁₋₄ tel que 2-hydroxyéthyle, 2-hydroxybutyle et 2-hydroxypropyle; ou un groupe alcoxy (C₁₋₄) alkyle (C₁₋₄) tel que méthoxyéthyle, éthoxyéthyle et méthoxypropyle,
- un groupe cycloalkyle en C₃ à C₁₂, tel que le groupe isobornyle,
- un groupe aryle en C₃ à C₂₀ tel que le groupe phényle,
- un groupe aralkyle en C₄ à C₃₀ (groupe alkyle en C₁ à C₈) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,
lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆, ou un groupe phényle.
- (iv) les composés vinyliques de formules :
CH₂=CH-R₉, CH₂=CH-CH₂-R₉ ou CH₂=C(CH₃)-CH₂-R₉
dans lesquelles R₉ est un groupe hydroxyle, halogène (Cl ou F), NH₂, OR₁₀ où R₁₀ représente un groupe phényle ou un groupe alkyle en C₁ à C₁₂ (le monomère est un éther de vinyle ou d'allyle); acétamide (NHCOCH₃); un groupe OCOR₁₁ où R₁₁ représente un groupe alkyle de 2 à 12 carbones, linéaire ou ramifié (le monomère est un ester de vinyle ou d'allyle) ; ou un groupe choisi parmi :
- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆ ou un groupe phényle ;
- un groupe cycloalkyle en C₃ à C₁₂ tel que isobornyle, cyclohexane,
- un groupe aryle en C₃ à C₂₀ tel que phényle,
- un groupe aralkyle en C₄ à C₃₀ (groupe alkyle en C₁ à C₈) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,
lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si(R₄R₅) où R₄ et R₅ identiques ou différents représentent un groupe alkyle en C₁ à C₆, ou un groupe phényle.
- (v) les monomères (méth)acryliques, (méth)acrylamides ou vinyliques à groupe fluoré ou perfluoré, tels que le (méth)acrylate d'éthyl-perfluorooctyle ou d'éthyl-2-perfluorohexyle;
- (vi) les monomères (méth)acryliques, (méth)acrylamides ou vinyliques siliconés, tels que le méthacryloxypropyltris(triméthylsiloxy)silane ou l'acryloxypropylpolydiméthylsiloxane.
- (vii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- (viii) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

17. Composition selon l'une des revendications 12 à 16, dans laquelle le ou les comonomères additionnels sont présents en une quantité de 30% à 99,99% en poids par rapport au poids du polymère final, notamment en une quantité de 50% à 99,9% en poids, en particulier de 70% à 99,5% en poids, voire de 80 à 99% en poids, encore mieux de 90 à 98% en poids.

18. Composition selon l'une des revendications 12 à 17, dans laquelle les comonomères additionnels sont choisis parmi, seuls ou en mélange, les (méth)acrylates d'alkyle en C1-C18 ou de cycloalkyle en C3-C12, et notamment parmi l'acrylate de méthyle, le méthacrylate de méthyle, l'acrylate d'isobornyle, le méthacrylate d'isobornyle, l'acrylate d'isobutyle, le méthacrylate d'isobutyle, l'acrylate d'éthyl-2-hexyle, le méthacrylate d'éthyl-2-hexyle, l'acrylate de dodécyle, le méthacrylate de dodécyle, l'acrylate de stéaryle, le méthacrylate de stéaryle, l'acrylate de trifluoroéthyle, le méthacrylate de trifluoroéthyle; ou encore l'acide acrylique, l'acide méthacrylique, le méthacryloxypropyltris(triméthylsiloxy)silane, l'acryloxypropyltris(triméthylsiloxy)silane, l'acryloxypropylpolydiméthylsiloxane et le méthacryloxypropylpolydiméthylsiloxane.

19. Composition selon l'une des revendications précédentes, dans laquelle le polymère présente une masse moléculaire moyenne en poids (Mw) comprise entre 5000 et 600 000 g/mol, notamment entre 10 000 et 300 000 g/mol, et encore mieux entre 20 000 et 150 000 g/mol.

20. Composition selon l'une des revendications précédentes, dans laquelle le polymère est présent, seul ou en mélange, en une quantité de 0,01 à 60% en poids, de préférence 0,1 à 50% en poids, notamment 1 à 25% en poids, voire 3 à 15% en poids, et encore mieux 5 à 12% en poids, par rapport au poids total de la composition.

21. Composition selon l'une des revendications précédentes, dans laquelle le milieu physiologiquement acceptable comprend un milieu hydrophile comprenant de l'eau ou un mélange eau/solvant(s) organique(s) hydrophile(s) et/ou comprend une phase grasse.

22. Composition selon l'une des revendications précédentes, dans laquelle la phase grasse comprend des cires, corps gras pâteux, gommes, solvants organiques lipophiles, huiles, et/ou de leurs mélanges.

23. Composition selon l'une des revendications précédentes, comprenant en outre une phase particulaire qui peut comprendre des pigments et/ou des nacres et/ou des charges.

24. Composition selon l'une des revendications précédentes, comprenant des matières colorantes choisies parmi les colorants hydrosolubles et/ou les colorants liposolubles.

25. Composition selon l'une des revendications précédentes, comprenant au moins un polymère additionnel tel qu'un polymère filmogène.

26. Composition selon l'une des revendications précédentes, comprenant au moins un ingrédient choisi parmi les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.

27. Composition selon l'une des revendications précédentes, se présentant sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre; une lotion, une crème, une pommade, une pâte souple, un onguent, un solide coulé ou moulé et notamment en stick ou en coupelle, ou encore de solide compacté.

28. Composition selon l'une des revendications précédentes, se présentant sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

29. Composition selon l'une des revendications précédentes, se présentant sous la forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cemes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux); d'une composition de protection ou de soin de la peau du visage, du cou, des mains ou du corps, notamment une composition anti-rides, une composition hydratante ou traitante; une composition anti-solaire ou de bronzage artificiel; d'un produit capillaire, notamment pour la coloration, le maintien de la coiffure, la mise en forme des cheveux, le soin, le traitement ou le nettoyage des cheveux, telle que des shampooings, des gels, des lotions de mise en plis, des lotions pour le brushing, des compositions de fixation et de coiffage telles que les laques ou spray.

30. Procédé cosmétique de maquillage ou de soin des matières kératiniques, notamment de la peau du corps ou du visage, des lèvres, des ongles, des cils, des sourcils et/ou des cheveux, comprenant l'application sur lesdites matières d'une composition cosmétique telle que définie à l'une des revendications 1 à 29.

## Claims

1. Cosmetic or pharmaceutical composition comprising, in a physiologically acceptable medium, at least one polymer comprising at least one monomeric compound of formula (I): 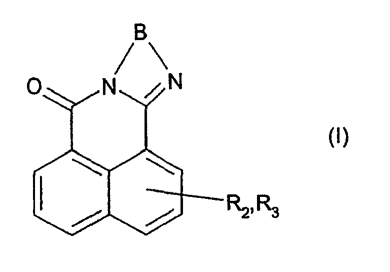 in which:
- R₂ and R₃, which are present on the same ring or each on a different ring, represent, independently of each other, a hydrogen, a halogen or a group of formula -X-G-P (II), with the proviso that at least one of the radicals R₂ and/or R₃ represents a group of formula (II), in which:
- X is chosen from the groups -O-, -S-, -SO-, -SO₂-, -NH- and -NR- with R representing a linear, branched and/or cyclic, saturated and/or unsaturated carbon-based radical containing 1 to 30 carbon atoms, optionally substituted with one or more groups chosen from =O, OH, NH₂ and halogen atoms; and/or optionally interrupted with one or more heteroatoms chosen from O, N, P, Si and S;
- G is a linear, branched and/or cyclic, saturated and/or unsaturated divalent carbon-based radical containing 1 to 32 carbon atoms, optionally substituted with one or more groups chosen from =O, OH, NH₂ and halogen atoms; and/or optionally interrupted with one or more heteroatoms chosen from O, N, P, Si and S;
- P is a polymerizable group chosen from one of the following formulae: 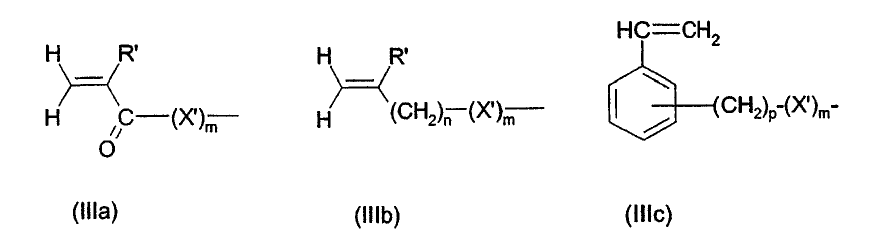 in which:
- R' represents H or a linear or branched, saturated C₁-₆ hydrocarbon-based radical,
- X' represents O, NH or NR" with R" representing a radical chosen from C₁-₆ alkyl, C₆-₁₀ aryl, (C₆-₁₀)-aryl(C₁-₆)alkyl and (C₁-₆) alkyl (C₆-₁₀) aryl radicals, the alkyl and/or aryl groups also possibly being substituted with one or more groups chosen from OH, halogen, C₁-₆ alkoxy and C₆-₁₀ aryloxy; preferably, X' represents 0;
- m is equal to 0 or 1; n is equal to 0 or 1; p is equal to 0, 1 or 2; and
- B represents one of the following divalent aromatic groups (IVa) to (IVd): 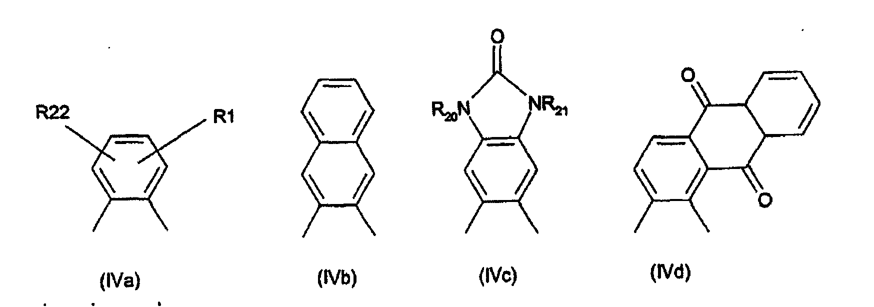 in which:
- R₁ is a linear, branched and/or cyclic, saturated and/or unsaturated carbon-based radical containing 1 to 32 carbon atoms, optionally substituted with one or more groups chosen from =O, OH, NH₂ and halogen atoms; and/or optionally interrupted with one or more heteroatoms chosen from O, N, P, Si and S;
- R₂₂ is a hydrogen atom or a linear, branched and/or cyclic, saturated and/or unsaturated carbon-based radical containing 1 to 32 carbon atoms, optionally substituted with one or more groups chosen from =O, OH, NH₂ and halogen atoms; and/or optionally interrupted with one or more heteroatoms chosen from O, N, P, Si and S;
- R₂₀ and R₂₁ are, independently of each other, a hydrogen atom, a linear or branched C₁₋₈ alkyl radical or a cyclopentyl, cyclohexyl, cyclooctyl, cyclodecyl, cyclododecyl, benzyl, naphthyl or phenyl radical.

2. Composition according to the preceding claim, in which, in the monomeric compound, the radical R₂ is a hydrogen atom and R₃ is a group of formula (II).

3. Composition according to either of the preceding claims, in which, in the monomeric compound, in the group of formula (II), X is chosen from -O-, -NH- and - NR- with R preferentially representing a linear, branched and/or cyclic, saturated or unsaturated hydrocarbon-based radical optionally comprising a hydrocarbon-based ring that is itself saturated or unsaturated, containing 2 to 18 and especially 3 to 12 carbon atoms, optionally substituted with one or more groups chosen from O0, OH, NH₂ and halogen atoms; and/or optionally interrupted with one or more heteroatoms chosen from O, N, P, Si and S.

4. Composition according to one of the preceding claims, in which, in the monomeric compound, X is chosen from -NH- and -NR- with R representing a cyclohexyl.

5. Composition according to one of the preceding claims, in which, in the monomeric compound, the divalent radical G is a linear, branched and/or cyclic, saturated or unsaturated divalent hydrocarbon-based radical optionally comprising a hydrocarbon-based ring that is itself saturated or unsaturated, containing in total 2 to 18 and especially 3 to 10 carbon atoms, optionally substituted with one or more groups chosen from =O, OH, NH₂ and halogen atoms; and/or optionally interrupted with one or more heteroatoms chosen from O, N, P and Si.

6. Composition according to one of the preceding claims, in which, in the monomeric compound, G is chosen from linear or branched, saturated divalent hydrocarbon-based radicals optionally comprising a saturated hydrocarbon-based ring, containing in total 2 to 18 and especially 3 to 10 carbon atoms.

7. Composition according to one of the preceding claims, in which, in the monomeric compound, the polymerizable group P is chosen from one of the following formulae: 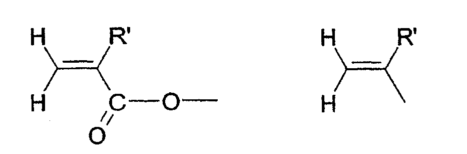 in which R' represents H or methyl.

8. Composition according to one of the preceding claims, in which, in the monomeric compound, the group B is chosen from those of formula (IVa) in which R₁ is preferentially a linear, branched and/or cyclic, saturated carbon-based radical containing 1 to 32 carbon atoms, especially 2 to 12 or even 3 to 6 carbon atoms; in particular, R₁ may be a methyl, ethyl or propyl radical.

9. Composition according to one of the preceding claims, in which the monomeric compound corresponds to one of the following formulae in which R is hydrogen or methyl:

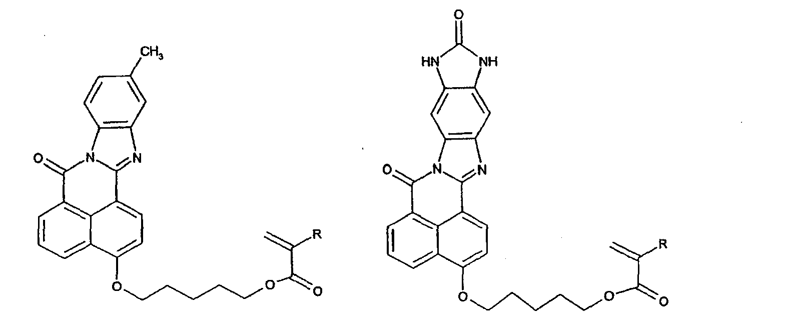

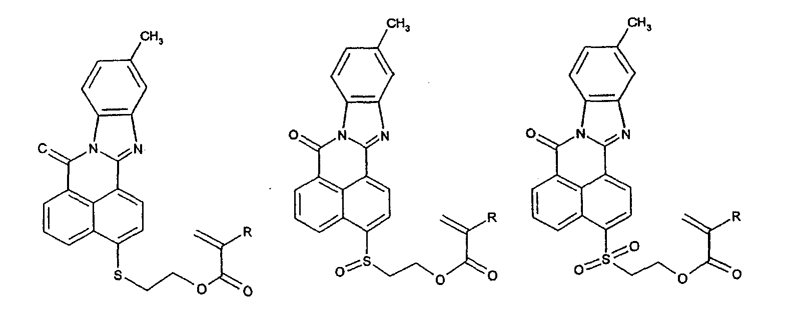

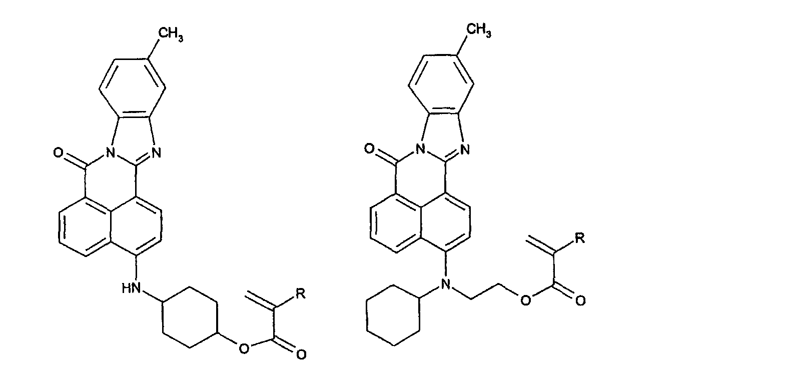

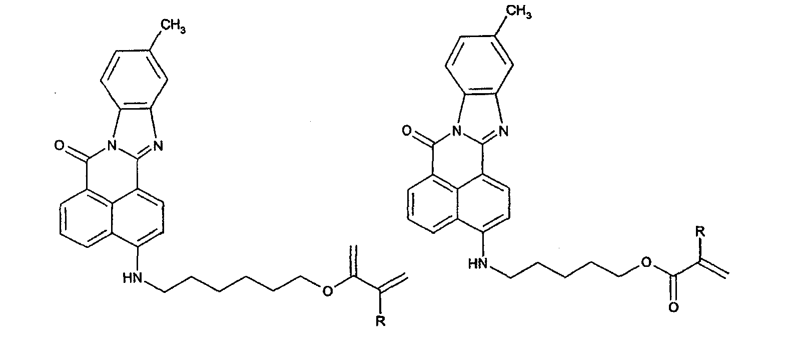

10. Composition according to one of the preceding claims, in which the polymer is a homopolymer of a monomeric compound as defined in one of Claims 1 to 9.

11. Composition according to one of Claims 1 to 9, in which the polymer is a copolymer comprising only monomeric compounds as defined in one of Claims 1 to 9.

12. Composition according to one of Claims 1 to 9, in which the polymer is a copolymer comprising at least one monomeric compound as defined in one of Claims 1 to 9, and at least one additional comonomer.

13. Composition according to either of Claims 11 and 12, in which the polymer is a statistical, alternating, grafted, block or gradient copolymer.

14. Composition according to either of Claims 11 and 12, in which the monomeric compound is present in an amount of from 0.01% to 70% by weight relative to the weight of said polymer, especially in an amount of from 0.1% to 50% by weight, in particular from 0.5% to 30% by weight, or even from 1% to 20% by weight and better still from 2% to 10% by weight, the additional comonomers, alone or as a mixture, representing the remainder to 100% by weight.

15. Composition according to one of Claims 12 to 14, in which the polymer comprises at least one additional comonomer with an optical effect chosen from the compounds of formula (A) and/or of formula (B) below: 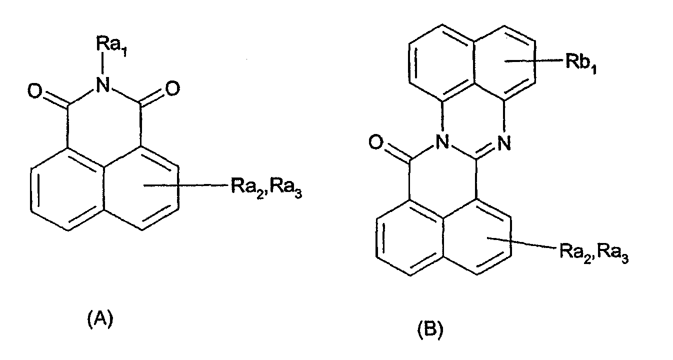 in which:
- Ra₁ represents a linear, branched and/or cyclic, saturated and/or unsaturated carbon-based radical containing 1 to 32 carbon atoms; optionally substituted with one or more groups chosen from =O, OH, NH₂ and halogen atoms; and/or optionally interrupted with one or more heteroatoms chosen from O, N, P, Si and S;
- Rb₁ is chosen from (i) a hydrogen atom, (ii) a halogen, (iii) a linear, branched and/or cyclic, saturated and/or unsaturated carbon-based radical containing 1 to 12 carbon atoms, optionally substituted with one or more groups chosen from =O, OH and NH₂ and/or optionally interrupted with one or more heteroatoms chosen from O, N, P, Si and S; (iv) a group NRR' with R and R' being, independently of each other, a hydrogen atom or a linear, cyclic or branched, saturated C₁₋₆ hydrocarbon-based radical, especially methyl, ethyl, propyl, isopropyl, n-butyl, isobutyl, tert-butyl, pentyl or hexyl;
- Ra₂ and Ra₃, which are present on the same ring or each on a different ring, represent, independently of each other, a hydrogen, a halogen or a group of formula -Xa-Ga-Pa (II), with the proviso that at least one of the radicals Ra₂ and/or Ra₃ represents a group of formula (II), in which:
- Xa is chosen from the groups -O-, -S-, -SO-, -SO₂-, -NH- and -NR₄- with R₄ representing a linear, branched and/or cyclic, saturated and/or unsaturated carbon-based radical containing 1 to 30 carbon atoms, optionally substituted with one or more groups chosen from =O, OH, NH₂ and halogen atoms; and/or optionally interrupted with one or more heteroatoms chosen from O, N, P, Si and S;
- Ga is a linear, branched and/or cyclic, saturated and/or unsaturated divalent carbon-based radical containing 1 to 32 carbon atoms, optionally substituted with one or more groups chosen from =O, OH, NH₂ and halogen atoms; and/or optionally interrupted with one or more heteroatoms chosen from O, N, P, Si and S;
- Pa is a polymerizable group chosen from one of the following formulae: 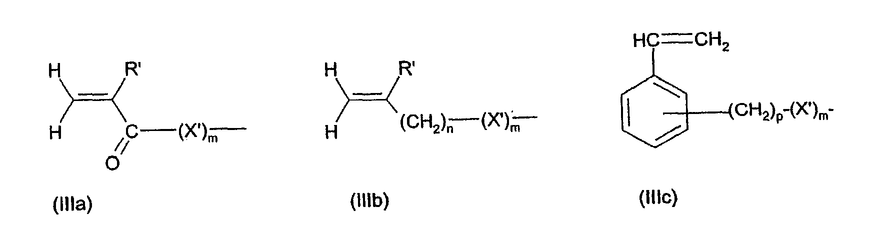 in which:
- R' represents H or a linear or branched, saturated C₁₋₆ hydrocarbon-based radical,
- X' represents O, NH or NR" with R" representing a radical chosen from C₁₋₆ alkyl, C₆₋₁₀ aryl, (C₆₋₁₀)aryl(C₁₋₆ )alkyl and (C₁₋₆) alkyl (C₆₋₁₀)aryl radicals, the alkyl and/or aryl groups also possibly being substituted with one or more groups chosen from OH, halogen, C₁₋₆ alkoxy and C₆₋₁₀ aryloxy; and
- m is equal to 0 or 1; n is equal to 0 or 1; p is equal to 0, 1 or 2.

16. Composition according to one of Claims 12 to 15, in which the polymer comprises at least one additional comonomer chosen, alone or as a mixture, from the following monomers:
- (i) ethylenic hydrocarbons containing from 2 to 10 carbons, such as ethylene, isoprene or butadiene;
- (ii) the (meth)acrylates of formula:
CH₂ = CHCOOR'₃
or 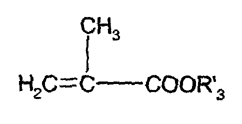 in which R'₃ represents:
- a linear or branched alkyl group of 1 to 18 carbon atoms, in which is (are) optionally intercalated one or more heteroatoms chosen from O, N, S and P; said alkyl group also possibly being optionally substituted with one or more substituents chosen from hydroxyl groups, halogen atoms (Cl, Br, I and F), and groups Si(R₄R₅), in which R₄ and R₅, which may be identical or different, represent a C₁ to C₆ alkyl group or a phenyl group; R'₃ may especially be a methyl, ethyl, propyl, n-butyl, isobutyl, tert-butyl, hexyl, ethylhexyl, octyl, lauryl, isooctyl, isodecyl, dodecyl, cyclohexyl, t-butylcyclohexyl or stearyl group; 2-ethylperfluorohexyl; or a C₁₋₄ hydroxyalkyl group such as 2-hydroxyethyl, 2-hydroxybutyl or 2-hydroxypropyl; or a (C₁₋₄)alkoxy(C₁₋₄)alkyl group such as methoxyethyl, ethoxyethyl or methoxypropyl,
- a C₃ to C₁₂ cycloalkyl group such as an isobornyl group,
- a C₃ to C₂₀ aryl group such as a phenyl group,
- a C₄ to C₃₀ aralkyl group (C₁ to C₈ alkyl group) such as 2-phenylethyl, t-butylbenzyl or benzyl,
- a 4- to 12-membered heterocyclic group containing one or more heteroatoms chosen from 0, N and S, the ring being aromatic or non-aromatic,
- a heterocycloalkyl group (1 to 4 C alkyl), such as furfurylmethyl or tetrahydrofurfurylmethyl,
said cycloalkyl, aryl, aralkyl, heterocyclic or heterocycloalkyl groups possibly being optionally substituted with one or more substituents chosen from hydroxyl groups, halogen atoms and linear or branched C₁₋₄ alkyl groups in which is (are) optionally intercalated one or more heteroatoms chosen from O, N, S and P, said alkyl groups also possibly being optionally substituted with one or more substituents chosen from hydroxyl groups, halogen atoms (Cl, Br, I and F), and groups Si(R₄R₅), in which R₄ and R₅, which may be identical or different, represent a C₁ to C₆ alkyl group or a phenyl group,
- R'₃ may also be a group -(C₂H₄O)ₘ-R", with m = 5 to 150 and R" = H or C₁ to C₃₀ alkyl, for example -POE-methyl or
- POE-behenyl;
- (iii) the (meth)acrylamides of formula: 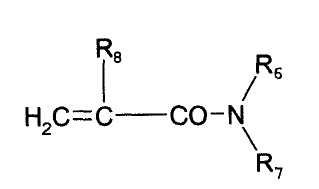 in which R₈ denotes H or methyl; and R₇ and R₆, which may be identical or different, represent:
- a hydrogen atom; or
- a linear or branched alkyl group of 1 to 18 carbon atoms, in which is (are) optionally intercalated one or more heteroatoms chosen from O, N, S and P; said alkyl group also possibly being optionally substituted with one or more substituents chosen from hydroxyl groups, halogen atoms (Cl, Br, I and F), and groups Si(R₄R₅), in which R₄ and R₅, which may be identical or different, represent a C₁ to C₆ alkyl group or a phenyl group; R₆ and/or R₇ may especially be a methyl, ethyl, propyl, n-butyl, isobutyl, tert-butyl, hexyl, ethylhexyl, octyl, lauryl, isooctyl, isodecyl, dodecyl, cyclohexyl, t-butylcyclohexyl or stearyl group; 2-ethylperfluorohexyl; or a C₁₋₄ hydroxyalkyl group such as 2-hydroxyethyl, 2-hydroxybutyl or 2-hydroxypropyl; or a (C₁₋₄)alkoxy(C₁₋₄)alkyl group such as methoxyethyl, ethoxyethyl or methoxypropyl,
- a C₃ to C₁₂ cycloalkyl group, such as an isobornyl group,
- a C₃ to C₂₀ aryl group such as a phenyl group,
- a C₄ to C₃₀ aralkyl group (C₁ to C₈ alkyl group) such as 2-phenylethyl, t-butylbenzyl or benzyl,
- a 4- to 12-membered heterocyclic group containing one or more heteroatoms chosen from 0, N and S, the ring being aromatic or non-aromatic,
- a heterocycloalkyl group (1 to 4 C alkyl), such as furfurylmethyl or tetrahydrofurfurylmethyl,
said cycloalkyl, aryl, aralkyl, heterocyclic or heterocycloalkyl groups possibly being optionally substituted with one or more substituents chosen from hydroxyl groups, halogen atoms and linear or branched C₁-C₄ alkyl groups in which is (are) optionally intercalated one or more heteroatoms chosen from O, N, S and P, said alkyl groups also possibly being optionally substituted with one or more substituents chosen from hydroxyl groups, halogen atoms (Cl, Br, I and F) and groups Si(R₄R₅), in which R₄ and R₅, which may be identical or different, represent a C₁ to C₆ alkyl group, or a phenyl group;
- (iv) the vinyl compounds of formulae: CH₂=CH-R₉, CH₂=CH-CH₂-R₉ or CH₂=C(CH₃)-CH₂-R₉
in which R₉ is a hydroxyl group, halogen (Cl or F), NH₂, OR₁₀ in which R₁₀ represents a phenyl group or a C₁ to C₁₂ alkyl group (the monomer is a vinyl or allylic ether); acetamide (NHCOCH₃); a group OCOR₁₁ in which R₁₁ represents a linear or branched alkyl group of 2 to 12 carbons (the monomer is a vinyl or allylic ester); or a group chosen from:
- a linear or branched alkyl group of 1 to 18 carbon atoms, in which is (are) optionally intercalated one or more heteroatoms chosen from O, N, S and P; said alkyl group also possibly being optionally substituted with one or more substituents chosen from hydroxyl groups, halogen atoms (Cl, Br, I and F) and groups Si(R₄R₅), in which R₄ and R₅, which may be identical or different, represent a C₁ to C₆ alkyl group or a phenyl group;
- a C₃ to C₁₂ cycloalkyl group such as isobornyl or cyclohexane,
- a C₃ to C₂₀ aryl group such as phenyl,
- a C₄ to C₃₀ aralkyl group (C₁ to C₈ alkyl group) such as 2-phenylethyl; benzyl,
- a 4- to 12-membered heterocyclic group containing one or more heteroatoms chosen from O, N and S, the ring being aromatic or non-aromatic,
- a heterocycloalkyl group (1 to 4 C alkyl), such as furfurylmethyl or tetrahydrofurfurylmethyl,
said cycloalkyl, aryl, aralkyl, heterocyclic or heterocycloalkyl groups possibly being optionally substituted with one or more substituents chosen from hydroxyl groups, halogen atoms and linear or branched 1 to 4 C alkyl groups in which is (are) optionally intercalated one or more heteroatoms chosen from O, N, S and P, said alkyl groups also possibly being optionally substituted with one or more substituents chosen from hydroxyl groups, halogen atoms (Cl, Br, I and F) and groups Si(R₄R₅) in which R₄ and R₅, which may be identical or different, represent a C₁ to C₆ alkyl group, or a phenyl group;
- (v) (meth)acrylic, (meth)acrylamide or vinyl monomers containing a fluoro or perfluoro group, such as ethylperfluorooctyl or 2-ethylperfluorohexyl (meth)acrylate;
- (vi) silicone-based (meth)acrylic, (meth)acrylamide or vinyl monomers, such as methacryloxypropyltris(trimethylsiloxy)silane or acryloxypropylpolydimethylsiloxane;
- (vii) ethylenically unsaturated monomers comprising at least one carboxylic, phosphoric or sulfonic acid, or anhydride, function, for instance acrylic acid, methacrylic acid, crotonic acid, maleic anhydride, itaconic acid, fumaric acid, maleic acid, acrylamidopropanesulfonic acid, vinylbenzoic acid and vinylphosphoric acid, and the salts thereof;
- (viii) ethylenically unsaturated monomers comprising at least one tertiary amine function, for instance 2-vinylpyridine, 4-vinylpyridine, dimethylaminoethyl methacrylate, diethylaminoethyl methacrylate or dimethylaminopropylmethacrylamide, and the salts thereof.

17. Composition according to one of Claims 12 to 16, in which the additional comonomer(s) is (are) present in an amount of from 30% to 99.99% by weight, especially in an amount of from 50% to 99.9% by weight, in particular from 70% to 99.5% by weight, or even from 80% to 99% by weight, and better still from 90% to 98% by weight, relative to the weight of the final polymer.

18. Composition according to one of Claims 12 to 17, in which the additional comonomers are chosen, alone or as a mixture, from C₁-C₁₈ alkyl or C₃-C₁₂ cycloalkyl (meth)acrylates, and especially from methyl acrylate, methyl methacrylate, isobornyl acrylate, isobornyl methacrylate, isobutyl acrylate, isobutyl methacrylate, 2-ethylhexyl acrylate, 2-ethylhexyl methacrylate, dodecyl acrylate, dodecyl methacrylate, stearyl acrylate, stearyl methacrylate, trifluoroethyl acrylate and trifluoroethyl methacrylate; or alternatively acrylic acid, methacrylic acid, methacryloxypropyltris(trimethylsiloxy)silane, acryloxypropyltris(trimethylsiloxy)silane, acryloxypropylpolydimethylsiloxane and methacryloxypropylpolydimethylsiloxane.

19. Composition according to one of the preceding claims, in which the polymer has a weight-average molecular mass (Mw) of between 5000 and 600 000 g/mol, especially between 10 000 and 300 000 g/mol and better still between 20 000 and 150 000 g/mol.

20. Composition according to one of the preceding claims, in which the polymer is present, alone or as a mixture, in an amount of from 0.01% to 60% by weight, preferably 0.1% to 50% by weight, especially 1% to 25% by weight or even 3% to 15% by weight and better still 5% to 12% by weight, relative to the total weight of the composition.

21. Composition according to one of the preceding claims, in which the physiologically acceptable medium comprises a hydrophilic medium comprising water or a water/hydrophilic organic solvent(s) mixture and/or comprises a fatty phase.

22. Composition according to one of the preceding claims, in which the fatty phase comprises waxes, pasty fatty substances, gums, lipophilic organic solvents and oils, and/or mixtures thereof.

23. Composition according to one of the preceding claims, also comprising a particulate phase that may comprise pigments and/or nacres and/or fillers.

24. Composition according to one of the preceding claims, comprising dyestuffs chosen from water-soluble dyes and/or liposoluble dyes.

25. Composition according to one of the preceding claims, comprising at least one additional polymer such as a film-forming polymer.

26. Composition according to one of the preceding claims, comprising at least one ingredient chosen from vitamins, thickeners, gelling agents, trace elements, softeners, sequestrants, fragrances, acidifying or basifying agents, preserving agents, sunscreens, surfactants, antioxidants, hair-loss counteractants, antidandruff agents, propellants and ceramides, or mixtures thereof.

27. Composition according to one of the preceding claims, which is in the form of a suspension, a dispersion especially of oil in water by means of vesicles; an optionally thickened or even gelled oily solution; an oil-in-water, water-in-oil or multiple emulsion; a gel or a mousse; an oily or emulsified gel; a dispersion of vesicles, especially lipid vesicles; a two-phase or multi-phase lotion; a spray; a loose, compact or cast powder; an anhydrous paste; a lotion, a cream, a pomade, a soft paste, an ointment, a cast or moulded solid especially as a stick or in a dish, or alternatively a compacted solid.

28. Composition according to one of the preceding claims, which is in the form of a care and/or makeup product for bodily or facial skin, the lips, the nails, the eyelashes, the eyebrows and/or the hair, an antisun or self-tanning product, or a hair product for caring for, treating, shaping, making up or dyeing the hair.

29. Composition according to one of the preceding claims, which is in the form of a makeup composition, especially a complexion product such as a foundation, a makeup rouge or an eyeshadow; a lip product such as a lipstick or a lipcare product; a concealer product; a blusher, a mascara or an eyeliner; an eyebrow makeup product, a lip pencil or an eye pencil; a nail product such as a nail varnish or a nailcare product; a body makeup product; a hair makeup product (hair mascara or hair lacquer); a composition for protecting or caring for the skin of the face, the neck, the hands or the body, especially an antiwrinkle composition or a moisturizing or treating composition; an antisun or artificial tanning composition; a hair product, especially for dyeing, holding the hairstyle, shaping the hair, caring for, treating or cleansing the hair, such as shampoos, hairsetting gels or lotions, blow-drying lotions, and fixing and styling compositions such as lacquers or sprays.

30. Cosmetic process for making up or caring for keratin materials, especially bodily or facial skin, the lips, the nails, the eyelashes, the eyebrows and/or the hair, comprising the application to said materials of a cosmetic composition as defined in any one of Claims 1 to 29.

## Patentansprüche

1. Kosmetische oder pharmazeutische Zusammensetzung, die in einem physiologisch akzeptablen Medium mindestens ein Polymer enthält, das mindestens eine monomere Verbindung der folgenden Formel (I) umfasst: 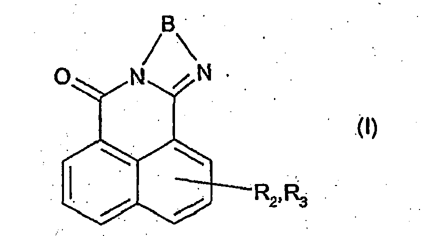 wobei in der Formel:
- die Gruppen R₂ und R₃, die an dem gleichen Ring oder jede an einem unterschiedlichen Ring vorliegen, bedeuten unabhängig voneinander Wasserstoff, ein Halogen oder eine Gruppe der Formel -X-G-P (II), mit der Maßgabe, dass mindestens eine der Gruppen R₂ und/oder R₃ eine Gruppe der Formel (II) bedeutet, in der:
- X unter den Gruppen -O-, -S-, -SO-, -SO₂-, -NH- oder -NRausgewählt ist, wobei R eine lineare, verzweigte und/oder cyclische, gesättigte und/oder ungesättigte Gruppe auf Kohlenstoffbasis mit 1 bis 30 Kohlenstoffatomen bedeutet, die gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P, Si und S ausgewählt sind;
- G eine lineare, verzweigte und/oder cyclische, gesättigte und/oder ungesättigte zweiwertige Gruppe auf Kohlenstoffbasis mit 1 bis 32 Kohlenstoffatomen bedeutet, die gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P, Si und S ausgewählt sind;
- P eine polymerisierbare Gruppe ist, die unter einer der folgenden Formeln ausgewählt ist: 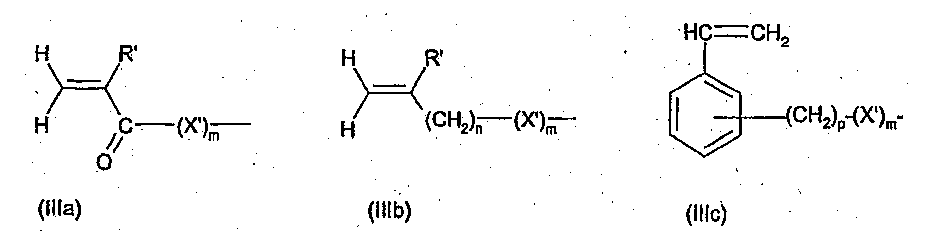 wobei in den Formeln bedeuten:
- R' bedeutet H oder eine lineare oder verzweigte, gesättigte Kohlenwasserstoffgruppe mit 1-6 C,
- X' bedeutet O, NH oder NR", wobei R" eine Gruppe ist, die unter den C₁₋₆-Alkylgruppen, C₆₋₁₀-Arylgruppen, Aryl(C₆₋₁₀)-alkyl(C₁₋₆)gruppen oder Alkyl(C₁₋₆)aryl(C₆₋₁₀)gruppen ausgewählt ist, wobei die Alkylgruppen und/oder Arylgruppen ferner mit einer oder mehreren Gruppen substituiert sein können, die unter OH, Halogen, C₁₋₆-Alkoxy und C₆₋₁₀-Aryloxy ausgewählt sind; vorzugsweise bedeutet X' O;
- m ist 0 oder 1; n ist 0 oder 1; p ist 0, 1 oder 2;
- B bedeutet eine der folgenden zweiwertigen aromatischen Gruppen (IVa) bis (IVd): 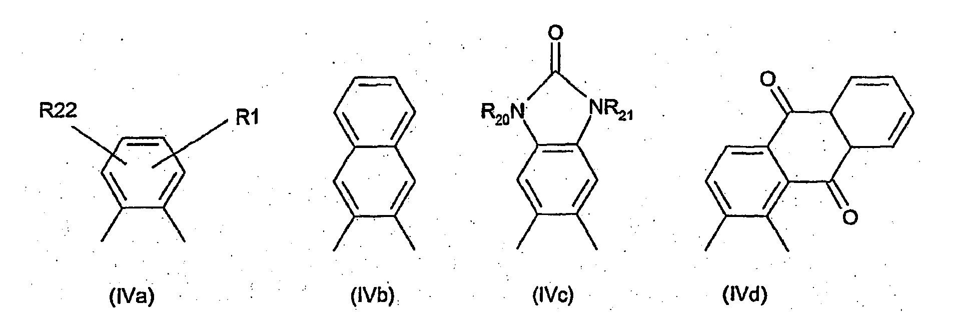 wobei in den Formeln:
- R1 ist eine lineare, verzweigte und/oder cyclische, gesättigte und/oder ungesättigte Gruppe auf Kohlenstoffbasis mit 1 bis 32 Kohlenstoffatomen, die gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P, Si und S ausgewählt sind;
- R22 ist ein Wasserstoffatom oder eine lineare, verzweigte und/oder cyclische, gesättigte und/oder ungesättigte Gruppe auf Kohlenstoffbasis mit 1 bis 32 Kohlenstoffatomen, die gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P, Si und S ausgewählt sind;
- R₂₀ und R₂₁ bedeuten unabhängig voneinander ein Wasserstoffatom, eine lineare oder verzweigte C₁₋₈-Alkylgruppe, eine Gruppe Cyclopentyl, Cyclohexyl, Cyclooctyl, Cyclodecyl, Cyclododecyl, Benzyl, Naphthyl oder Phenyl.

2. Zusammensetzung nach einem der vorhergehenden Ansprüche, bei der in der monomeren Verbindung die Gruppe R₂ ein Wasserstoffatom ist und die Gruppe R₃ eine Gruppe der Formel (II) bedeutet.

3. Zusammensetzung nach einem der vorhergehenden Ansprüche, bei der in der monomeren Verbindung in der Gruppe der Formel (II) X unter -O-, -NH- und -NR- ausgewählt ist, wobei R vorzugsweise eine lineare, verzweigte und/oder cyclische, gesättigte oder ungesättigte Kohlenwasserstoffgruppe bedeutet, die gegebenenfalls einen gesättigten oder ungesättigten Kohlenwasserstoffring aufweist, die 2 bis 18 und insbesondere 3 bis 12 Kohlenstoffatome aufweist und gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P, Si und S ausgewählt sind.

4. Zusammensetzung nach einem der vorhergehenden Ansprüche, bei der in der monomeren Verbindung X unter -NH- und -NRausgewählt ist, wobei R Cyclohexyl bedeutet.

5. Zusammensetzung nach einem der vorhergehenden Ansprüche, bei der in der monomeren Verbindung die zweiwertige Gruppe G eine lineare, verzweigte und/oder cyclische, gesättigte oder ungesättigte zweiwertige Kohlenwasserstoffgruppe ist, die gegebenenfalls einen gesättigten oder ungesättigten Kohlenwasserstoffring aufweist, die insgesamt 2 bis 18, insbesondere 3 bis 10 Kohlenstoffatome aufweist und die gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P und Si ausgewählt sind.

6. Zusammensetzung nach einem der vorhergehenden Ansprüche, bei der in der monomeren Verbindung die Gruppe G unter den linearen oder verzweigten, gesättigten zweiwertigen Kohlenwasserstoffgruppen ausgewählt ist, die gegebenenfalls einen gesättigten Kohlenwasserstoffring aufweisen, mit insgesamt 2 bis 18 und insbesondere 3 bis 10 Kohlenstoffatomen.

7. Zusammensetzung nach einem der vorhergehenden Ansprüche, bei der in der monomeren Verbindung die polymerisierbare Gruppe P unter einer der folgenden Formeln ausgewählt ist: 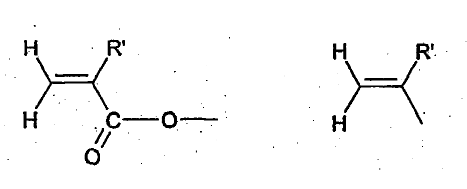 wobei R' H oder Methyl bedeutet.

8. Zusammensetzung nach einem der vorhergehenden Ansprüche, bei der in der monomeren Verbindung die Gruppe B unter den Gruppen der Formel (IVa) ausgewählt ist, in der R₁ vorzugsweise eine lineare, verzweigte und/oder cyclische, gesättigte Gruppe auf Kohlenstoffbasis mit 1 bis 32, insbesondere 2 bis 12 und sogar 3 bis 6 Kohlenstoffatomen bedeutet; wobei R₁ insbesondere eine Gruppe Methyl, Ethyl oder Propyl sein kann.

9. Zusammensetzung nach einem der vorhergehenden Ansprüche, bei der die monomere Verbindung eine der folgenden Formeln entspricht, bei denen R Wasserstoff oder Methyl bedeutet:

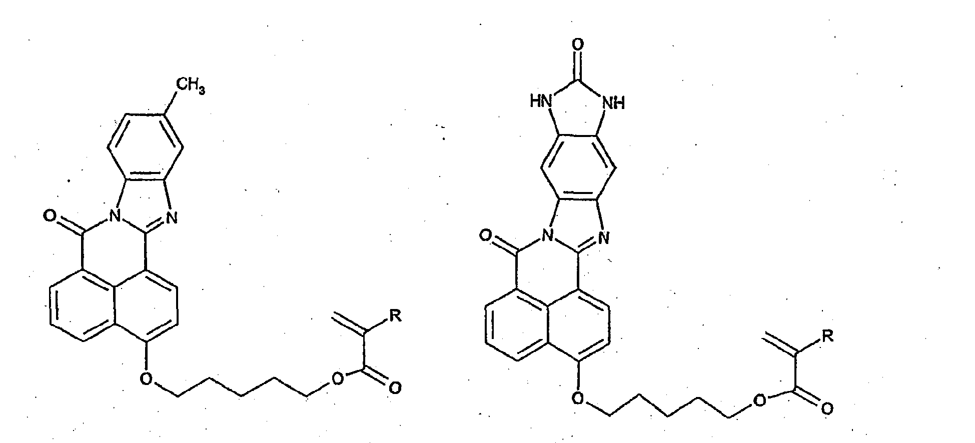

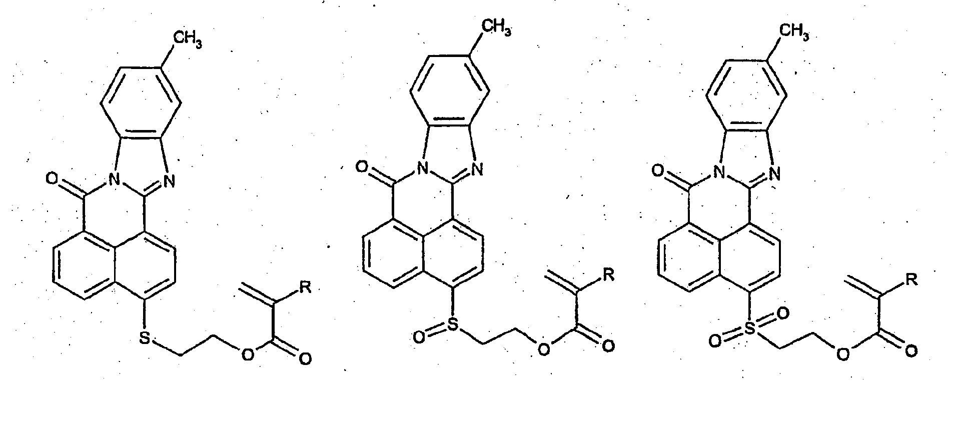

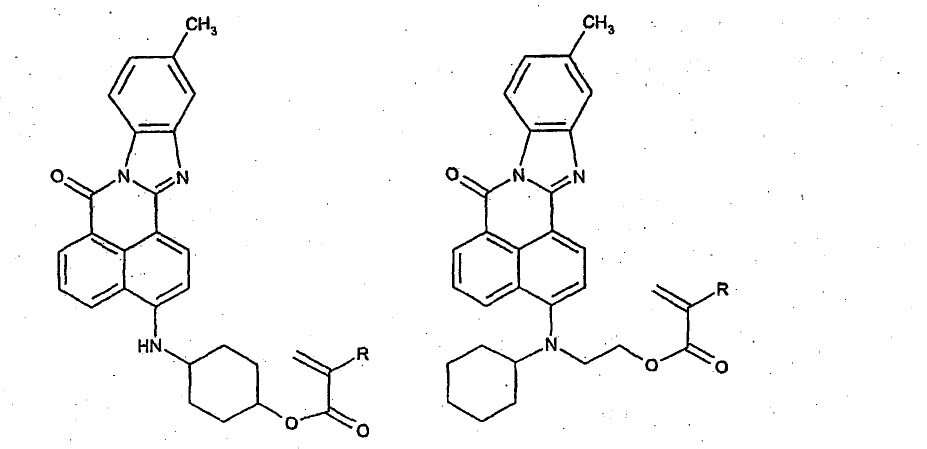

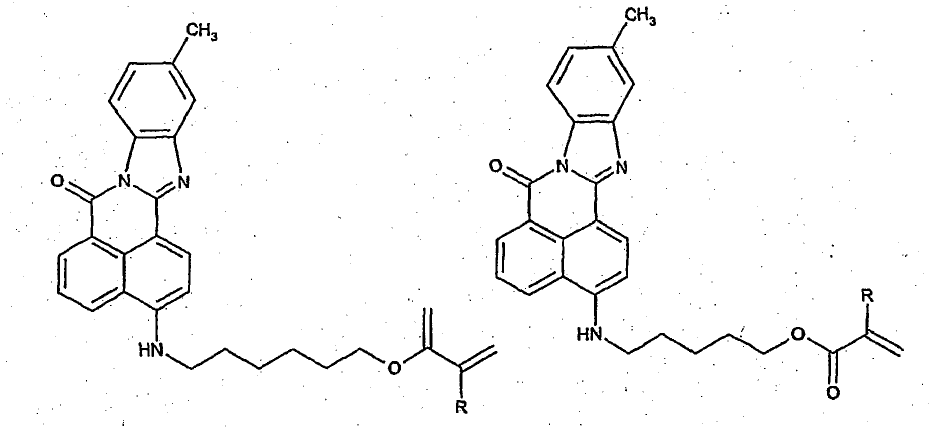

10. Zusammensetzung nach einem der vorhergehenden Ansprüche, bei der das Polymer ein Homopolymer einer monomeren Verbindung, wie sie in einem der Ansprüche 1 bis 9 definiert ist, bedeutet.

11. Zusammensetzung nach einem der Ansprüche 1 bis 9, bei der das Polymer ein Copolymer ist, das nur monomere Verbindungen aufweist, wie sie in einem der Ansprüche 1 bis 9 definiert sind.

12. Zusammensetzung nach einem der Ansprüche 1 bis 9, bei der das Polymer ein Copolymer ist, das mindestens eine monomere Verbindung, wie sie in einem der Ansprüche 1 bis 9 definiert ist, und mindestens ein zusätzliches Comonomer enthält.

13. Zusammensetzung nach einem der Ansprüche 11 bis 12, bei der das Polymer ein statistisches Copolymer, alternierendes Copolymer, gepfropftes Copolymer, Sequenzcopolymer oder Gradientencopolymer ist.

14. Zusammensetzung nach einem der Ansprüche 11 bis 12, bei der die monomere Verbindung in einer Menge von 0,01 bis 70 Gew.-%, bezogen auf das Gewicht des Polymers, insbesondere in einer Menge von 0,1 bis 50 Gew.-%, besonders 0,5 bis 30 Gew.-%, sogar 1 bis 20 Gew.-%, noch besser 2 bis 10 Gew.-% vorliegt, wobei die zusätzlichen Copolymere einzeln oder als Gemisch die für 100 Gew.-% erforderliche Restmenge ausmachen.

15. Zusammensetzung nach einem der Ansprüche 12 bis 14, bei der das Polymer mindestens ein zusätzliches Comonomer mit optischem Effekt enthält, das unter den Verbindungen der folgenden Formeln (A) und/oder der Formel (B) ausgewählt ist: 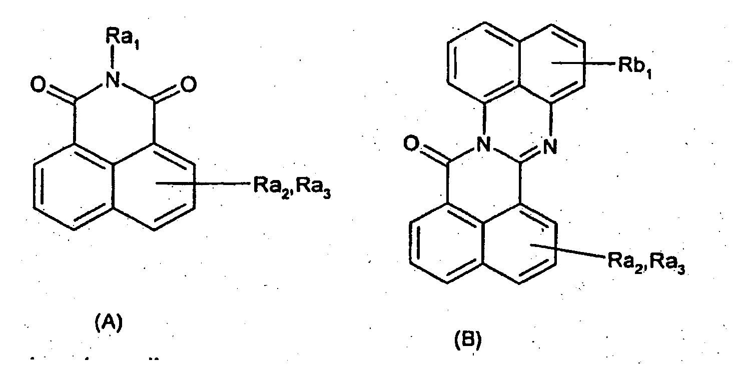 in der Formel:
- Ra₁ bedeutet eine lineare, verzweigte und/oder cyclische, gesättigte und/oder ungesättigte Gruppe auf Kohlenstoffbasis mit 1 bis 32 Kohlenstoffatomen; die gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P, Si und S ausgewählt sind;
- Rb₁ ist ausgewählt unter (i) einem Wasserstoffatom, (ii) einem Halogen, (iii) einer linearen, verzweigten und/oder cyclischen, gesättigten und/oder ungesättigten Gruppe auf Kohlenstoffbasis mit 1 bis 12 Kohlenstoffatomen, die gegebenenfalls mit einer oder mehreren Gruppen substituiert ist, die unter =O, OH, NH₂ ausgewählt sind, und/oder gegebenenfalls durch ein oder mehrere Heteroatome unterbrochen ist, die unter O, N, P, Si und S ausgewählt sind; (iv) einer Gruppe NRR', wobei R und R' unabhängig voneinander ein Wasserstoffatom oder eine lineare, cyclische oder verzweigte, gesättigte C₁₋₆-Kohlenwasserstoffgruppe und insbesondere Methyl, Ethyl, Propyl, Isopropyl, *n*-Butyl, Isobutyl, Isobutyl, *tert*-Butyl, Pentyl oder Hexyl bedeuten;
- Ra₂ und Ra₃, die an dem gleichen Ring oder jede an einem unterschiedlichen Ring vorhanden sind, bedeuten unabhängig voneinander ein Wasserstoffatom, ein Halogen oder eine Gruppe der Formel -Xa-Ga-Pa (II), mit der Maßgabe, dass mindestens eine der Gruppen Ra₂ und/oder Ra₃ eine Gruppe der Formel (II) bedeutet, worin:
- Xa ausgewählt ist unter den Gruppen -O-, -S-, -SO-, -SO₂-, -NH- oder -NR₄, wobei R₄ eine lineare, verzweigte und/oder cyclische, gesättigte und/oder ungesättigte Gruppe auf Kohlenstoffbasis mit 1 bis 30 Kohlenstoffatomen bedeutet, die gegebenenfalls mit einer oder mehreren Gruppen substituiert ist, die unter =O, OH, NH₂ und den Halogenatomen ausgewählt ist; und/ oder gegebenenfalls durch ein oder mehrere Heteroatome unterbrochen ist, die unter O, N, P, Si und S ausgewählt sind;
- Ga ist eine lineare, verzweigte und/oder cyclische, gesättigte und/oder ungesättigte zweiwertige Gruppe auf Kohlenstoffbasis mit 1 bis 32 Kohlenstoffatomen, die gegebenenfalls mit einer oder mehreren Gruppen substituiert ist, die unter =O, OH, NH₂ und den Halogenatomen ausgewählt sind; und/oder gegebenenfalls durch ein oder mehrere Heteroatome unterbrochen ist, die unter O, N, P, Si und S ausgewählt sind;
- Pa ist eine polymerisierbare Gruppe, die unter einer der folgenden Formeln ausgewählt ist: 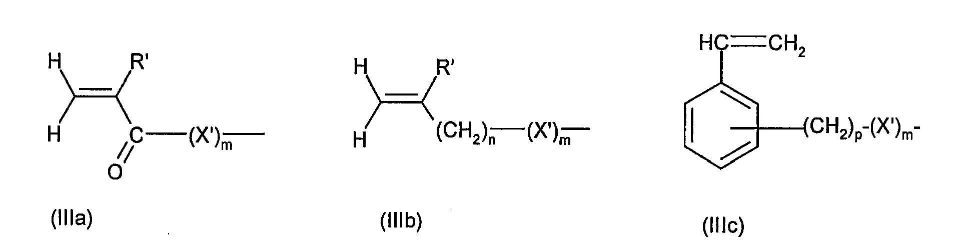 wobei in den Formeln:
- R' bedeutet H oder eine lineare oder verzweigte, gesättigte C₁₋₆-Kohlenwasserstoffgruppe,
- X' bedeutet O, NH oder NR", wobei R" eine Gruppe ist, die unter den Alkyl(C₁₋₆)gruppen, Aryl(C₆₋₁₀)gruppen, Aryl(C₆₋₁₀)alkyl(C₁₋₆)gruppen oder Alkyl(C₁₋₆)aryl(C₆₋₁₀)gruppen ausgewählt ist, wobei die Alkylgruppen und/oder Arylgruppen ferner mit einer oder mehreren Gruppen substituiert sein können, die unter OH, Halogen, Alkoxy(C₁₋₆) und Aryloxy(C₆₋₁₀) ausgewählt sind; und
- m bedeutet 0 oder 1; n ist 0 oder 1; p ist 0, 1 oder 2.

16. Zusammensetzung nach einem der Ansprüche 12 bis 15, bei der das Polymer mindestens ein ergänzendes Comonomer aufweist, das einzeln oder in Form eines Gemisches unter den folgenden Monomeren ausgewählt ist:
- (i) ethylenischen Kohlenwasserstoffen mit 2 bis 10 Kohlenstoffatomen, wie Ethylen, Isopren oder Butadien;
- (ii) (Meth)acrylaten der Formel:
CH₂ = CHCOOR'₃
oder 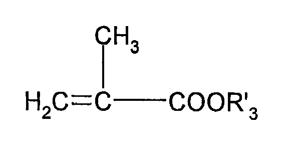 wobei in den Formeln R'₃ bedeutet:
- eine lineare oder verzweigte Alkylgruppe mit 1 bis 18 Kohlenstoffatomen, bei der gegebenenfalls ein oder mehrere Heteroatome eingebaut ist (sind), die unter O, N, S und P ausgewählt sind; wobei die Alkylgruppe ferner gegebenenfalls mit einem oder mehreren Substituenten substituiert sein kann, die unter den Hydroxylgruppen, Halogenatomen (Cl, Br, I und F) und den Gruppen Si(R₄R₅) ausgewählt sind, wobei R₄ und R₅, die gleich oder verschieden sind, eine C₁₋₆-Alkylgruppe oder eine Phenylgruppe bedeuten; wobei R'₃ insbesondere bedeuten kann: eine Gruppe Methyl, Ethyl, Propyl, *n*-Butyl, Isobutyl, *tert*-Butyl, Hexyl, Ethylhexyl, Octyl, Lauryl, Isooctyl, Isodecyl, Dodecyl, Cyclohexyl, *tert*-Butylcyclohexyl oder Stearyl; 2-Ethylperfluorhexyl oder eine C₁₋₄-Hydroxyalkylgruppe, wie 2-Hydroxyethyl, 2-Hydroxybutyl und 2-Hydroxypropyl; oder eine Alkoxy(C₁₋₄)alkyl(C₁₋₄)-gruppe, wie Methoxyethyl, Ethoxyethyl und Methoxypropyl,
- eine C₃₋₁₂-Cycloalkylgruppe, wie beispielsweise Isobornyl,
- eine C₃₋₂₀-Arylgruppe, wie beispielsweise die Phenylgruppe,
- eine C₄₋₃₀-Aralkylgruppe (C₁₋₈-Alkylgruppe), wie 2-Phenylethyl, *tert*-Butylbenzyl oder Benzyl,
- eine heterocyclische Gruppe mit 4 bis 12 Verknüpfungen, die ein oder mehrere Heteroatome enthält, die unter O, N und S ausgewählt sind, wobei der Ring aromatisch oder nichtaromatisch ist,
- eine Heterocycloalkylgruppe (Alkyl mit 1 bis 4 C), wie Furfurylmethyl oder Tetrahydrofurfurylmethyl,
wobei die Cycloalkylgruppen, Arylgruppen, Aralkylgruppen, heterocyclischen Gruppen oder Heterocycloalkylgruppen gegebenenfalls mit einem oder mehreren Substituenten substituiert sein können, die unter den Hydroxygruppen, Halogenatomen und linearen oder verzweigten C₁₋₄-Alkylgruppen ausgewählt sind, bei denen gegebenenfalls ein oder mehrere Heteroatome eingebaut ist (sind), die unter O, N, S und P ausgewählt sind, wobei die Alkylgruppen ferner gegebenenfalls mit einem oder mehreren Substituenten substituiert sein können, die unter den Hydroxygruppen, Halogenatomen (Cl, Br, I und F) und den Gruppen Si(R₄R₅) ausgewählt sind, wobei die Gruppen R₄ und R₅, die gleich oder verschieden sind, eine C₁₋₆-Alkylgruppe oder eine Phenylgruppe bedeuten,
- R'₃ kann auch eine Gruppe -(C₂H₄O)ₘ-R" sein, mit m = 5 bis 150 und R' = H oder C₁₋₃₀-Alkyl, beispielsweise -PEO-Methyl oder -PEO-Behenyl;
- (iii) (Meth)acrylamiden der Formel: 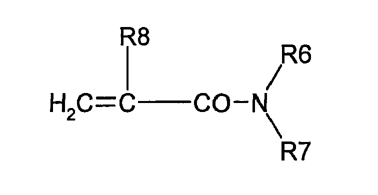 wobei R₈ H oder Methyl bedeutet; und R₇ und R₆, die identisch oder verschieden sind, bedeuten:
- ein Wasserstoffatom; oder
- eine lineare oder verzweigte Alkylgruppe mit 1 bis 18 Kohlenstoffatomen, bei der gegebenenfalls ein oder mehrere Heteroatome eingebaut ist (sind), die unter O, N, S und P ausgewählt sind; wobei die Alkylgruppe ferner gegebenenfalls mit einem oder mehreren Substituenten substituiert sein kann, die unter den Hydroxygruppen, Halogenatomen (Cl, Br, I und F) und den Gruppen Si(R₄R₅) ausgewählt sind, wobei die Gruppen R₄ und R₅, die gleich oder verschieden sind, eine C₁₋₆-Alkylgruppe oder eine Phenylgruppe bedeuten; wobei R₆ und/oder R₇ insbesondere bedeuten können; eine Gruppe Methyl, Ethyl, Propyl, *n*-Butyl, Isobutyl, *tert*-Butyl, Hexyl, Ethylhexyl, Octyl, Lauryl, Isooctyl, Isodecyl, Dodecyl, Cyclohexyl, *tert*-Butylcyclohexyl oder Stearyl; 2-Ethylperfluorhexyl; oder eine C₁₋₄-Hydroxyalkylgruppe, wie 2-Hydroxyethyl, 2-hydroxybutyl und 2-Hydroxypropyl; oder eine Alkoxy-(C₁₋₄)alkyl(C₁₋₄)gruppe, wie Methoxyethyl, Ethoxyethyl und Methoxypropyl.
- eine Cycloalkylgruppe mit 3 bis 12 Kohlenstoffatomen, wie die Gruppe Isobornyl,
- eine C₃₋₂₀-Arylgruppe, wie die Gruppe Phenyl,
- eine C₄₋₃₀-Aralkylgruppe (C₁₋₈-Alkylgruppe), beispielsweise 2-Phenylethyl, *tert*-Butylbenzyl oder Benzyl,
- eine heterocyclische Gruppe mit 4 bis 12 Bestandteilen, die ein oder mehrere Heteroatome aufweist, die unter O, N und S ausgewählt sind, wobei der Ring aromatisch oder nichtaromatisch ist,
- eine Heterocycloalkylgruppe (Alkyl mit 1 bis 4 C), wie Furfurylmethyl oder Tetrahydrofurfurylmethyl,
wobei die Cycloalkylgruppen, Arylgruppen, Aralkylgruppen, heterocyclischen Gruppen oder Heterocycloalkylgruppen gegebenenfalls mit einem oder mehreren Substituenten substituiert sein können, die unter den Hydroxygruppen, Halogenatomen und linearen oder verzweigten Alkylgruppen mit 1 bis 4 Kohlenstoffatomen ausgewählt sind, bei denen gegebenenfalls ein oder mehrere Heteroatome eingebaut ist (sind), die unter O, N, S und P ausgewählt sind, wobei die Alkylgruppen ferner gegebenenfalls mit einem oder mehreren Substituenten substituiert sein können, die unter den Hydroxygruppen, Halogenatomen (Cl, Br, I und F) und den Gruppen Si(R₄R₅) ausgewählt sind, wobei die Gruppen R₄ und R₅, die gleich oder verschieden sind, eine C₁₋₆-Alkylgruppe oder eine Phenylgruppe bedeuten,
- (iv) Vinylverbindungen der Formeln:
CH₂=CH-R₉, CH₂=CH-CH₂-R₉ oder CH₂=C(CH₃)-CH₂-R₉, bei denen Rg bedeutet: eine Hydroxygruppe, Halogen (Cl oder F), NH₂, OR₁₀, wobei R₁₀ eine Phenylgruppe oder eine C₁₋₁₂ -Alkylgruppe ist (das Monomer ist ein Vinylether oder Allylether); Acetamid (NHCOCH₃); eine Gruppe OCOR₁₁, wobei R₁₁ eine Alkylgruppe mit 2 bis 12 Kohlenstoffatomen bedeutet, die linear oder verzweigt ist (das Monomer ist ein Vinylester oder Allylester); oder eine Gruppe, die ausgewählt ist unter:
- einer linearen oder verzweigten Alkylgruppe mit 1 bis 18 Kohlenstoffatomen, bei der gegebenenfalls ein oder mehrere Heteroatome eingebaut ist (sind), die unter O, N, S und P ausgewählt sind; wobei die Alkylgruppe ferner gegebenenfalls mit einem oder mehreren Substituenten substituiert sein kann, die unter den Hydroxygruppen, Halogenatomen (Cl, Br, I und F) und den Gruppen Si(R₄R₅) ausgewählt sind, wobei R₄ und R₅, die identisch oder voneinander verschieden sind, eine C₁₋₆-Alkylgruppe
oder eine Phenylgruppe bedeuten;
- einer C₃₋₁₂-Cycloalkylgruppe, wie Isobornyl, Cyclohexan,
- einer C₃₋₂₀-Arylgruppe, wie Phenyl,
- einer C₄₋₃₀-Aralkylgruppe (Alkyl mit C1-8), wie 2-Phenylethyl; Benzyl,
- einer heterocyclischen Gruppe mit 4 bis 12 Bestandteilen, die ein oder mehrere Heteroatome aufweist, die unter O, N und S ausgewählt sind, wobei der Ring aromatisch oder nichtaromatisch ist,
- einer Hetercycloalkylgruppe (Alkyl mit 1 bis 4 C), wie Furfurylmethyl oder Tetrahydrofurfurylmethyl, wobei die Cycloalkylgruppen, Arylgruppen, Aralkylgruppen, heterocyclischen Gruppen oder Heterocycloalkylgruppen gegebenenfalls mit einem oder mehreren Substituenten substituiert sein können, die unter den Hydroxygruppen, Halogenatomen und linearen oder verzweigten C₁₋₄-Alkylgruppen ausgewählt sind, bei denen gegebenenfalls ein oder mehrere Heteroatome eingebaut ist (sind), die unter O, N, S und P ausgewählt sind, wobei die Alkylgruppen ferner gegebenenfalls mit einem oder mehreren Substituenten substituiert sein können, die unter den Hydroxygruppen, Halogenatomen (Cl, Br, I und F) und den Gruppen Si(R₄R₅) ausgewählt sind, wobei die Gruppen R₄ und R₅, die gleich oder verschieden sind, eine C₁₋₆-Alkylgruppe oder eine Phenylgruppe bedeuten;
- (v) (Meth)acrylmonomeren, (Meth)acrylamidmonomeren oder Vinylmonomeren mit fluorierter oder perfluorierter Gruppe, wie beispielsweise Ethylperfluoroctyl(meth)acrylat oder 2-Ethylperfluorhexyl(meth)acrylat;
- (vi) (Meth)acrylmonomeren, (Meth)acrylamidmonomeren oder Vinylmonomeren, die siliconiert sind, wie Methacryloxypropyltris(trimethylsiloxy)silan oder Acryloxypropylpolydimethylsiloxan;
- (vii) Monomeren mit einer oder mehreren ethylenisch ungesättigten Bindungen, die mindestens eine Carbonsäurefunktion, Phosphorsäurefunktion oder Sulfonsäurefunktion oder Anhydridfunktion aufwiesen, wie beispielsweise Acrylsäure, Methacrylsäure, Crotonsäure, Maleinsäureanhydrid, Itaconsäure, Fumarsäure, Maleinsäure, Acrylamidopropansulfonsäure, Vinylbenzoesäure, Vinylphosphorsäure und den Salzen dieser Verbindungen;
- (viii) Monomeren mit einer oder mehreren ethylenisch ungesättigten Bindungen, die mindestens eine tertiäre Aminfunktion aufweisen, wie 2-Vinylpyridin, 4-Vinylpyridin, Dimethylaminoethylmethacrylat, Diethylaminoethylmethacrylat, Dimethylaminopropylmethacrylamid und den Salzen dieser Verbindungen.

17. Zusammensetzung nach einem der Ansprüche 12 bis 16, bei der das oder die zusätzlichen Comonomere in einer Menge von 30 bis 99,99 Gew.-%, bezogen auf das Gewicht des fertigen Polymers, insbesondere in einer Menge von 50 bis 99,9 Gew.-%, besonders 70 bis 99,5 Gew.-%, sogar 80 bis 99 Gew.-% und noch besser 90 bis 98 Gew.-% vorhanden sind.

18. Zusammensetzung nach einem der Ansprüche 12 bis 17, bei der die zusätzlichen Comonomere einzeln oder als Gemisch ausgewählt sind unter Alkyl(C₁₋₁₈)(meth)acrylaten oder Cycloalkyl-(C₃₋₁₂)(meth)acrylaten und insbesondere Methylacrylat, Methylmethacrylat, Isobornylacrylat, Isobornylmethacrylat, Isobutylacrylat, Isobutylmethacrylat, 2-Ethylhexylacrylat, 2-Ethylhexylmethacrylat, Dodecylacrylat, Dodecylmethacrylat, Stearylacrylat, Stearylmethacrylat, Trifluorethylacrylat, Trifluorethylmethacrylat; oder auch Acrylsäure, Methacrylsäure, Methacryloxypropyltris(trimethylsiloxy)silan, Acryloxypropyltris(trimethylsiloxy)silan, Acryloxypropylpolydimethylsiloxan und Methacryloxypropylpolydimethylsiloxan.

19. Zusammensetzung nach einem der vorhergehenden Ansprüche, bei der das Polymer eine gewichtsmittlere Molmasse (Mw) aufweist, die im Bereich von 5 000 bis 600 000 g/mol, insbesondere 10 000 bis 300 000 g/mol und noch besser 20 000 bis 150 000 g/mol liegt.

20. Zusammensetzung nach einem der vorhergehenden Ansprüche, bei der das Polymer einzeln oder als Gemisch in einer Menge von 0,01 bis 60 Gew.-%, vorzugsweise 0,1 bis 50 Gew.-%, insbesondere 1 bis 25 Gew.-%, sogar 3 bis 15 Gew.-% und noch besser 5 bis 12 Gew.-%, bezogen auf das Gesamtgewicht der Zusammensetzung, enthalten ist.

21. Zusammensetzung nach einem der vorhergehenden Ansprüche, bei der das physiologisch akzeptable Medium ein hydrophiles Medium umfasst, das Wasser oder ein Gemisch Wasser/hydrophile(s) organische(s) Lösungsmittel enthält, und/oder eine Fettphase aufweist.

22. Zusammensetzung nach einem der vorhergehenden Ansprüche, bei der die Fettphase Wachse, pastöse Fettsubstanzen, Gummis, lipophile organische Lösungsmittel, Öle und/oder deren Gemische enthält.

23. Zusammensetzung nach einem der vorhergehenden Ansprüche, die ferner eine Partikelphase aufweist, die Pigmente und/oder Perlglanzpigmente und/oder Füllstoffe enthalten kann.

24. Zusammensetzung nach einem der vorhergehenden Ansprüche, die Farbmittel enthält, die unter den wasserlöslichen Farbstoffen und/oder fettlöslichen Farbstoffen ausgewählt sind.

25. Zusammensetzung nach einem der vorhergehenden Ansprüche, die mindestens ein ergänzendes Polymer enthält, beispielsweise ein filmbildendes Polymer.

26. Zusammensetzung nach einem der vorhergehenden Ansprüche, die mindestens einen Bestandteil enthält, der unter den Vitaminen, Verdickungsmitteln, Gelbildnern, Spurenelementen, beruhigenden Stoffen, Maskierungsmitteln, Parfums, Alkalisierungsmitteln oder Ansäuerungsmitteln, Konservierungsmitteln, Sonnenschutzfiltern, grenzflächenaktiven Stoffen, Antioxidantien, Wirkstoffen gegen Haarausfall, Antischuppenmitteln, Treibmitteln, Ceramiden oder deren Gemischen ausgewählt ist.

27. Zusammensetzung nach einem der vorhergehenden Ansprüche, die als Suspension, Dispersion, insbesondere von Öl in Wasser mit Hilfe von Vesikeln; ölige Lösung, die gegebenenfalls eingedickt oder sogar geliert ist; Öl-in-Wasser-Emulsion, Wasser-in-Öl-Emulsion oder multiple Emulsion; Gel oder Schaum; öliges oder emulgiertes Gel; Vesikeldispersion, insbesondere Lipidvesikeldispersion; zweiphasige oder mehrphasige Lotion; Spray; loses, kompaktiertes oder gegossenes Pulver; wasserfreie Paste; Lotion, Creme, Pomade, weiche Paste, Salbe, gegossener oder geformter Feststoff, insbesondere als Stift oder in Tiegeln, oder auch kompaktierter Feststoff vorliegt.

28. Zusammensetzung nach einem der vorhergehenden Ansprüche, die in Form eines Produktes für die Pflege und/oder zum Schminken der Haut des Körpers oder des Gesichts, der Lippen, der Nägel, der Wimpern, der Augenbrauen und/oder der Haare, als Sonnenschutzprodukt oder Selbstbräunungsprodukt, als haarkosmetisches Produkt für die Pflege, Behandlung, Formgebung, zum Schminken oder zum Färben der Haare vorliegt.

29. Zusammensetzung nach einem der vorhergehenden Ansprüche, die als Zusammensetzung zum Schminken, insbesondere als Produkt für den Teint, wie als Make-up, Lidschatten oder Wangenrouge; als Produkt für die Lippen, beispielsweise als Lippenstift oder Lippenpflege; als Produkt gegen Augenringe; Blush, Mascara, Eyeliner; als Produkt zum Schminken der Augenbrauen, Lippen, Crayon oder Crayon für die Augen; Produkt für die Nägel, wie beispielsweise als Nagellack oder Nagelpflege; als Produkt zum Schminken des Körpers; als Produkt zum Schminken der Haare (Mascara oder Lack für die Haare); als Zusammensetzung zum Schutz oder für die Pflege der Gesichtshaut, der Haut des Halses, der Hände oder des Körpers, insbesondere als Zusammensetzung gegen Falten, hydratisierende oder behandelnde Zusammensetzung; Zusammensetzung zum Sonnenschutz oder für die künstliche Bräunung; als haarkosmetisches Produkt, insbesondere zum Färben, für die Festigung der Frisur, der Formgebung der Haare, die Pflege, die Behandlung oder Reinigung der Haare, beispielsweise als Haarwaschmittel, Gel, Lotion für Wasserwellen, Lotion zum Fönen, festigende Zusammensetzung und frisurgebende Zusammensetzung, wie Lack oder Spray vorliegt.

30. Kosmetisches Verfahren zum Schminken oder für die Pflege von Keratinsubstanzen, insbesondere die Haut des Körpers oder des Gesichts, der Lippen, der Nägel, der Wimpern, der Augenbrauen und/oder der Haare, das umfasst, auf diese Keratinsubstanzen eine kosmetische Zusammensetzung aufzubringen, wie sie in einem der Ansprüche 1 bis 29 definiert ist.
